# EUROPÄISCHE PATENTSCHRIFT

(11) **EP 1 572 708 B1**
(45) Veröffentlichungstag und Bekanntmachung des Hinweises auf die Patenterteilung: **19.04.2006**
(21) Anmeldenummer: 03782250.9
(22) Anmeldetag: 28.11.2003
(51) Int. Cl.: C07H 17/00, C07H 17/02, A61K 31/70, A61P 3/10

(54) **NEUE HETEROCYCLISCHE FLUORGLYKOSIDDERIVATE, DIESE VERBINDUNGEN ENTHALTENDE ARZNEIMITTEL UND DEREN VERWENDUNG**
NOVEL FLUOROGLYCOSIDE HETEROCYCLIC DERIVATIVES, PHARMACEUTICAL PRODUCTS CONTAINING SAID COMPOUNDS AND THE USE THEREOF
NOUVEAUX DERIVES DE FLUORGLYCOSIDE HETEROCYCLIQUES, PRODUITS PHARMACEUTIQUES CONTENANT CES COMPOSES ET LEUR UTILISATION

(30) Priorität: 12.12.2002 DE 10258008
(43) Veröffentlichungstag der Anmeldung: 14.09.2005
(73) Patentinhaber: Sanofi-Aventis Deutschland GmbH, 65929 Frankfurt am Main (DE)
(72) Erfinder: FRICK, Wendelin, 65510 Hünstetten-Beuerbach (DE); GLOMBIK, Heiner, 65719 Hofheim (DE); KRAMER, Werner, 55130 Mainz-Laubenheim (DE); HEUER, Hubert, 55270 Schwabenheim (DE); BRUMMERHOP, Harm, 60316 Frankfurt (DE); PLETTENBURG, Oliver, 65795 Hattersheim (DE)
(86) Internationale Anmeldenummer: PCT/EP2003/013455
(87) Internationale Veröffentlichungsnummer: WO 2004/052903

(56) Entgegenhaltungen:
- EP-A- 0 850 948
- EP-A- 1 213 296
- WO-A-01/27128

## Beschreibung

Die Erfindung betrifft substituierte heterocyclische Fluorglykosidderivate, deren physiologisch verträgliche Salze sowie physiologisch funktionelle Derivate.

In der Literatur sind bereits mehrere Substanzklassen mit SGLT-Wirkung bekannt. All diesen Strukturen diente als Leitbild der Naturstoff Phlorizin. Von diesem wurden folgende Klassen abgeleitet, die in den nachfolgenden Schutzrechten beschrieben sind:
- Propiophenonglykoside von Tanabe (WO 0280936, WO 0280935, JP 2000080041 und EP 850948)
- 2-(Glucopyranoslyoxy)-benzylbenzole von Kissei (WO 0244192, WO 0228872 und WO 0168660)
- Glucopyranosyloxy-pyrazole von Kissei und Ajinomoto (WO 0268440, WO 0268439, WO 0236602 und WO 0116147)
- O-Glykosidbenzamide von Bristol-Myers Squibb (WO 0174835 und WO 0174834)
- und C-Arylglykoside von Bristol-Myers Squibb (WO 0127128 und US 2002137903).

Alle bekannten Strukturen enthalten als sehr wichtiges Strukturelement die Glucose.

Der Erfindung lag die Aufgabe zugrunde, neue Verbindungen zur Verfügung zu stellen, mit denen eine Prävention und Behandlung von Diabetes Typ 1 und Typ 2 möglich ist. Wir haben nun überraschenderweise gefunden, dass heterocyclische Fluorglykosidderivate die Wirkung auf SGLT steigen. Diese Verbindungen eignen sich daher besonders zur Prävention und Behandlung von Diabetes Typ 1 und Typ 2.

Die Erfindung betrifft daher Verbindungen der Formel I, 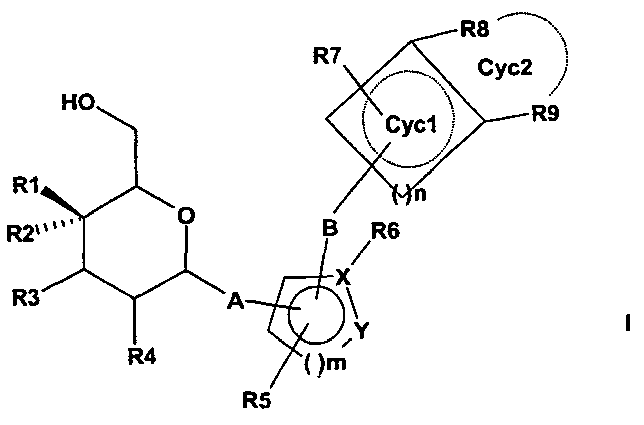 worin bedeuten
- R1 und R2: unabhängig voneinander F, H oder einer der Reste R1 oder R2 OH;
- R3: OH oder F, wobei mindestens einer der Reste R1, R2, R3 F sein muss;
- R4: OH;
- A: O, NH, CH₂, S oder eine Bindung;
- X: C, O, S oder N, wobei im Falle Y = O oder S, X = C sein müss;
- Y: N, O oder S;
- m: eine Zahl 1 oder 2;
- R5: Wasserstoff, F, Cl, Br, J, OH, CF₃, NO₂, CN, COOH, CO(C₁-C₆)-Alkyl, COO(C₁-C₆)-Alkyl, CONH₂, CONH(C₁-C₆)-Alkyl, CON[(C₁-C₆)-Alkyl]₂, (C₁₋C₆)-Alkyl, (C₂-C₆)-Alkenyl, (C₂-C₆)-Alkinyl, (C₁-C₆)-Alkoxy, HO-(C₁-C₆)- Alkyl, (C₁-C₆)-Alkyl-O-(C₁-C₆)-alkyl, Phenyl, Benzyl, (C₁-C₆)-Alkoxycarboxyl, wobei in den Alkyl-, Alkoxy-, Alkenyl- bzw. Alkinylresten ein, mehrere, oder alle Wasserstoff(e) durch Fluor ersetzt sein können; SO₂-NH₂, SO₂NH(C₁-C₆)-Alkyl, SO₂N[(C₁-C₆)-Alkyl]₂, S-(C₁-C₆)-Alkyl, S-(CH₂)ₒ-Pheny), SO-(C₁-C₆)-Alkyl, SO-(CH₂)ₒ-Phenyl, SO₂(C₁-C₆)-Alkyl, SO₂-(CH₂)ₒ-Phenyl, wobei o = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH(C₁-C₇)-Acyl. Phenyl, O-(CH₂)ₒ-Phenyl, wobei o = 0 - 6 sein kann, wobei der Phenylring ein bis 3-fach substituiert sein kann mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂₋CH₃, COOH, COO-(C₁-C₆)-Alkyl, CONH₂;
oder im Falle Y = S R5 und R6 gemeinsam mit den sie tragenden C-Atomen Phenyl;
- R6: gegebenenfalls H, (C₁-C₆)-Alkyl, (C₁-C₆)-Alkenyl, (C₃-C₆)-Cycloalkyl, oder Phenyl, dass gegebenenfalls durch Halogen oder (C₁-C₄)-Alkyl substituiert sein kann;
- B: (C₀-C₁₅)-Alkandiyl, wobei ein oder mehrere C-Atome des Alkandiyl-Rests unabhängig voneinander durch -O-, -(C=O)-, -CH=CH-, -C≡C-, -S-, -CH(OH)-, -CHF-, -CF₂-, -(S=O)-, -(SO₂)-, -N((C₁-C₆)-Alkyl)-, -N((C₁-C₆)-Alkyl-Phenyl)- oder -NH- ersetzt sein können;
- n: eine Zahl von 0 bis 4;
- Cyc1: ein 3 bis 7 gliedriger gesättigter, teilweise gesättigter oder ungesättigter Ring, wobei 1 C-Atom durch O, N oder S ersetzt sein kann;
- R7, R8, R9: Wasserstoff, F, Cl, Br, J, OH, CF₃, NO₂, CN, COOH, COO(C₁-C₆)-Alkyl, CO(C₁-C₄)-Alkyl, CONH₂, CONH(C₁-C₆)-Alkyl, CON[(C₁-C₆)-Alkyl]₂, (C₁- C₆)-Alkyl, (C₂-C₆)-Alkenyl, (C₂-C₆)-Alkinyl, (C₁-C₈)-Alkoxy, HO-(C₁-C₆)-Alkyl, (C₁-C₆)-Alkyl-O-(C₁-C₆)-alkyl, wobei in den Alkyl-, Alkoxy-, Alkeriyl-bzw. Alkinylresten ein, mehrere, oder alle Wasserstoff(e) durch Fluor ersetzt sein können;
SO₂-NH₂, SO₂NH(C₁-C₆)-Alkyl, SO₂N[(C₁-C₆)-Alkyl]₂, S-(C₁-C₆)-Alkyl, S-(CH₂)ₒ-Phenyl, SCF₃, SO-(C₁-C₆)-Alkyl, SO-(CH₂)ₒ-Phenyl, SO₂-(C₁-C₆)-Alkyl, SO₂-(CH₂)ₒ-Phenyl, wobei o = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH(C₁-C₇)-Acyl, Phenyl, O-(CH₂)ₒ-Phenyl, wobei o = 0 - 6 sein kann, wobei der Phenylring ein bis 3-fach substituiert sein kann mit F, Cl, Br, J, OH, CF₃, NO₂, CN, OCF₃, (C₁₋C₈)-Alkoxy, (C₁-C₆)-Alkyl, NH₂, NH(C₁-C₆)-Alkyl, N((C₁-C₆)-Alkyl)₂, SO₂₋CH₃, COOH, COO-(C₁-C₆)-Alkyl, CONH₂;
oder
- R8 und R9: gemeinsam mit den sie tragenden C-Atomen ein 5 bis 7 gliedrigen, gesättigter, teilweise oder vollständig ungesättigter Ring Cyc2, wobei 1 oder 2 C-Atom(e) des Ringes auch durch N, O oder S ersetzt sein können und Cyc2 gegebenenfalls durch (C₁-C₆)-Alkyl, (C₂-C₅)-Alkenyl, (C₂-C₅)-Alkinyl, wobei jeweils eine CH₂-Gruppe durch O ersetzt sein kann, oder durch H, F, Cl, OH, CF₃, NO₂, CN, COO(C₁-C₄)-Alkyl, CONH₂, CONH(C₁₋C₄)-Alkyl, OCF₃ substituiert sein kann;
sowie deren pharmazeutisch verträglichen Salze.

Die Verknüpfungspunkte von A, B und R5 an den Ring sind frei wählbar. Alle resultierenden Verbindungen der Formel I gehören zur vorliegenden Erfindung.

Als Heterocyclen des Zentralbausteines enthaltend X und Y kommen in Betracht: Thiophen, Furan, Pyrrol, Pyrazol, Isoxazol und Isothiazol, bevorzugt sind Thiophen, Pyrazol und Isoxazol. Besonders bevorzugt sind die Verbindungen der Formel I, die als Zentralbaustein Thiophen oder Pyrazol enthalten.

Bevorzugt sind Verbindungen der Formel I, worin bedeuten
- R1 und R2: unabhängig voneinander F oder H und einer der Reste R1 oder R2 = OH, wobei einer der Reste R1oder R2 F sein muss;
- R3: OH;
- R4: OH;
- A: O oder NH;
- X: C, O oder N, wobei im Falle Y = S, X = C sein muss;
- Y: S oder N;
- m: eine Zahl 1 oder 2;
- R5: Wasserstoff, F, Cl, Br, J, OH, CF₃, NO₂, CN, COOH, CO(C₁-C₆)-Alkyl, COO(C₁-C₆)-Alkyl, CONH₂, CONH(C₁-C₆)-Alkyl, CON[(C₁-C₆)-Alkyl]₂, (C₁₋C₆)-Alkyl, (C₂-C₆)-Alkenyl, (C₂-C₆)-Alkinyl, (C₁-C₆)-Alkoxy, HO-(C₁-C₆)-Alkyl, (C₁-C₆)-Alkyl-O-(C₁-C₆)-alkyl, Phenyl, Benzyl, (C₁-C₄)-Alkylcarboxyl, SO-(C₁-C₆)-Alkyl, wobei in den Alkyl- oder Alkoxyresten ein, mehrere, oder alle Wasserstoff(e) durch Fluor ersetzt sein können; oder
im Falle Y = S R5 und R6 gemeinsam mit den sie tragenden C-Atomen Phenyl;
- R6: gegebenenfalls H, (C₁-C₆)-Alkyl, (C₁-C₆)-Alkenyl, (C₃-C₆)-Cycloalkyl, oder Phenyl, das gegebenenfalls durch Halogen oder (C₁-C₄)-Alkyl substituiert sein kann;
- B: (C₀-C₁₅)-Alkandiyl, wobei ein oder mehrere C-Atom(e) des Alkandiyl-Rests unabhängig voneinander durch -O-, -(C=O)-, -CH=CH-, -C≡C-, -S-, -CH(OH)-, -CHF-, -CF₂-, -(S=O)-, -(SO₂)-, -N((C₁-C₆)-Alkyl)-, -N((C₁-C₆)-Alkyl-Phenyl)- oder -NH- ersetzt sein können;
- n: eine Zahl von 0 bis 4;
- Cyc1: ein 3 bis 7 gliedriger gesättigter, teilweise gesättigter oder ungesättigter Ring, wobei 1 C-Atom durch O oder S ersetzt sein kann;
- R7, R8, R9: Wasserstoff, F, Cl, Br, J, OH, CF₃, NO₂, CN, COOH, COO(C₁-C₆)-Alkyl, CO(C₁-C₄)-Alkyl, CONH₂, CONH(C₁-C₆)-Alkyl, CON[(C₁-C₆)-Alkyl]₂, (C₁₋C₆)-Alkyl, (C₂-C₆)-Alkenyl, (C₂-C₆)-Alkinyl, (C₁-C₈)-Alkoxy, HO-(C₁-C₆)-Alkyl, (C₁-C₆)-Alkyl-O-(C₁-C₆)-alkyl, S-(C₁-C₆)-Alkyl, SCF₃, SO-(C₁-C₆)-Alkyl, wobei in den Alkyl- bzw. Alkoxyresten ein, mehrere, oder alle Wasserstoff(e) durch Fluor ersetzt sein können;
oder
- R8 und R9: gemeinsam mit den sie tragenden C-Atomen ein 5 bis 7 gliedrigen, gesättigter, teilweise oder vollständig ungesättigter Ring Cyc2, wobei 1 oder 2 C-Atom(e) des Ringes auch durch N, O oder S ersetzt sein können und Cyc2 gegebenenfalls durch (C₁-C₆)-Alkyl, (C₂-C₅)-Alkenyl, (C₂-C₅)-Alkinyl, wobei jeweils eine CH₂-Gruppe durch O ersetzt sein kann, oder durch H, F, Cl, OH, CF₃, NO₂, CN, COO(C₁-C₄)-Alkyl, CONH₂, CONH(C₁₋C₄)-Alkyl, OCF₃ substituiert sein kann.

Bevorzugt sind ferner Verbindungen der Formel I, in denen die Zucker-Reste beta(β)-verknüpft sind und die Stereochemie in 2-, 3- und 5-Position des Zuckerrestes D-gluco-konfiguriert ist.

Besonders bevorzugt sind Verbindungen der Formel I, in den die Substituenten A und B eine benachbarte Stellung (ortho-Stellung) einnehmen.

Besonders bevorzugt sind Verbindungen der Formel I, worin
- R1 und R2: unabhängig voneinander F, H oder einer der Reste R1 oder R2 = OH, wobei mindestens einer der Reste R1 oder R2 F sein muss;
- R3: OH;
- R4: OH;
- A: O;
- X: C, O oder N, wobei im Falle Y = S, X = C sein muss;
- Y: S oder N;
- m: eine Zahl 1;
- R5: Wasserstoff, (C₁-C₅)-Alkyl, (C₁-C₄)-Alkoxy, HO-(C₁-C₄)-Alkyl, (C₁-C₄)-Alkyl-O-(C₁-C₄)-alkyl, F, Cl, CF₃, OCF₃, OCH₂CF₃ (C₁-C₄)-Alkyl-CF₂-, Phenyl, Benzyl, (C₁-C₄)-Alkylcarboxyl, (C₂-C₄)-Alkenyl, (C₂-C₄)-Alkinyl, COO(C₁-C₄)-Alkyl; oder
im Falle Y = S R5 und R6 gemeinsam mit den sie tragenden C-Atomen Phenyl;
- R6: gegebenenfalls H, (C₁-C₆)-Alkyl, (C₁-C₆)-Alkenyl, (C₃-C₆)-Cycloalkyl, oder Phenyl, dass gegebenenfalls durch Halogen oder (C₁-C₄)-Alkyl substituiert sein kann;
- B: (C₁-C₄)-Alkandiyl, wobei eine CH₂-Gruppe auch ersetzt sein kann durch -(C=O)-, -CH(OH)-, -CO-NH-, -CHF-, -CF₂-, -O-;
- n: eine Zahl 2 oder 3;
- Cyc1: ungesättigter 5 bis 6 gliedriger Ring, wobei 1 C-Atom durch O oder S ersetzt sein kann;
- R7,R8,R9: Wasserstoff, (C₁-C₄)-Alkyl, (C₁-C₈)-Alkoxy, S-(C₁-C₄)-Alkyl, SCF₃, F, Cl, Br, J, OCF₃, OCH₂CF₃, OH, HO-(C₁-C₄)-Alkyl, (C₁-C₄)-Alkyl-O-(C₁-C₄)-alkyl, oder
- R8 und R9: gemeinsam -CH=CH-O-, -CH=CH-S-, -CH=CH-CH=CH-, das gegebenenfalls durch (C₁-C₄)-Alkoxy substituiert ist, oder -O-(CH₂)ₚ-O-, mit p = 1 oder 2 und
- R7: Wasserstoff bedeuten.

Ganz besonders bevorzugt sind Verbindungen der Formel I, worin
- R1, R2: H oder F, wobei einer der Reste R1, R2 F sein muss;
- R3: OH;
- R4: OH;
- A: O;
- X: C und Y=S, oder
- X: O und Y = N, oder
- X: N und Y = N;
- m: eine Zahl 1;
- R5: Wasserstoff, CF₃, (C₁-C₆)-Alkyl, oder im Falle Y = S R5 und R6 gemeinsam mit den sie tragenden C-Atomen Phenyl;
- R6: gegebenenfalls H, (C₁-C₄)-Alkyl oder Phenyl;
- B: -CH₂-, -C₂H₄-, -C₃H₆, -CO-NH-CH₂- oder -CO-CH₂-CH₂-;
- n: eine Zahl 2 oder 3;
- Cyc1: ungesättigter 5 bis 6 gliedriger Ring, wobei 1 C-Atom durch S ersetzt sein kann;
- R7,R8,R9: Wasserstoff, (C₁-C₆)-Alkyl, (C₁-C₄)-Alkoxy, S-(C₁-C₄)-Alkyl, SCF₃, F, Cl, Br, J, OCF₃ oder
- R8 und R9: gemeinsam -CH=CH-O-, -CH=CH-CH=CH-, das gegebenenfalls durch (C₁-C₄)-Alkoxy substituiert ist, und
- R7: Wasserstoff bedeuten.

Ferner sind ganz besonders bevorzugt Verbindungen der Formel I, worin
- R1,R2: H oder F, wobei einer der Reste R1 oder R2 F bedeutet;
- R3: OH;
- R4: OH;
- A: O;
- X: C und Y = S oder
- X: NundY=N;
- m: eine Zahl 1;
- R5: Wasserstoff, (C₁-C₄)-Alkyl oder CF₃ oder im Falle Y = S R5 und R6 gemeinsam mit den sie tragenden C-Atomen Phenyl;
- R6: gegebenenfalls H oder (C₁-C₄)-Alkyl;
- B: -CH₂- oder -CO-NH-CH₂-;
- n: eine Zahl 2 oder 3;
- Cyc1: Phenyl oder Thiophen;
- R7: Wasserstoff, Methoxy, F, Cl, Br, J, (C₁-C₄)-Alkyl, OCF₃;
- R8,R9: Wasserstoff oder Cl oder
- R8 und R9: zusammen mit den sie tragenden C-Atomen Phenyl, das gegebenenfalls durch Methoxy substituiert sein kann, oder Furan und
- R7: Wasserstoff bedeuten.

Die Verknüpfung eines der Substituenten A oder B erfolgt besonders bevorzugt in Nachbarstellung zur Variablen Y.

Außerdem seien als ganz besonders bevorzugte Verbindungen solche genannt, in denen Y = S ist und solche, in denen R1 = H und R2 = F sind.

Die Erfindung bezieht sich auf Verbindungen der Formel I, in Form ihrer Racemate, racemischen Mischungen und reinen Enantiomere sowie auf ihre Diastereomere und Mischungen davon.

Die Alkylreste in den Substituenten R4, R5, R6, R7, R8 und R9 können sowohl geradkettig wie verzweigt sein. Unter Halogen werden F, Cl, Br, J, bevorzugt F und Cl verstanden.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, lsethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze), Erdalkalisalze (wie Magnesium- und Calciumsalze), Trometamol (2-Amino-2-hydroxymethyl-1,3-propandiol), Diethanolamin, Lysin oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen, wie zum Beispiel in H. Okada et al., Chem. Pharm. Bull. 1994,42, 57-61 beschrieben. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I wie vorstehend beschrieben, sowie ihre Salze, Solvate und physiologisch funktionellen Derivate wie hierin beschrieben.

Die Verbindung(en) der Formel (I) können auch in Kombination mit weiteren Wirkstoffen verabreicht werden.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrötransport oder lontophorese freigesetzt werden.

Gegenstand der Erfindung sind weiterhin Verfahren zur Herstellung der Verbindungen der allgemeinen Formel I, die entsprechend den folgenden Reaktionsschemata der Verfahren A, B und C erhalten werden können:

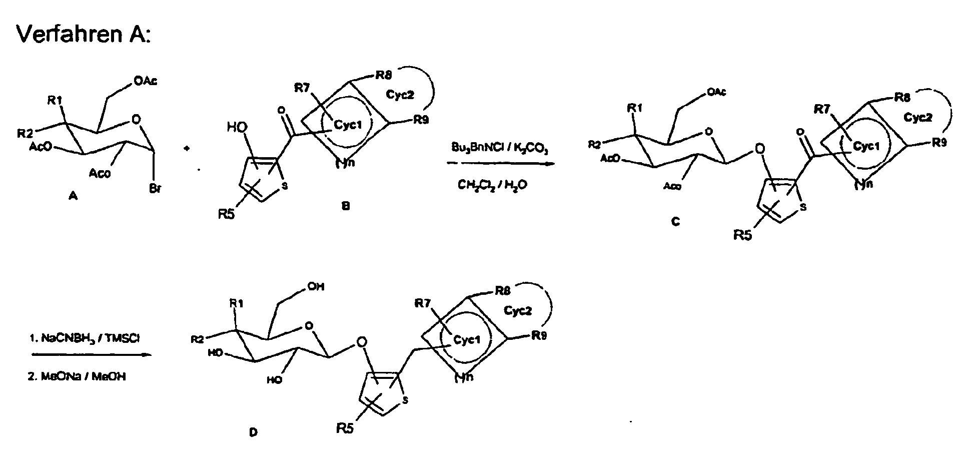

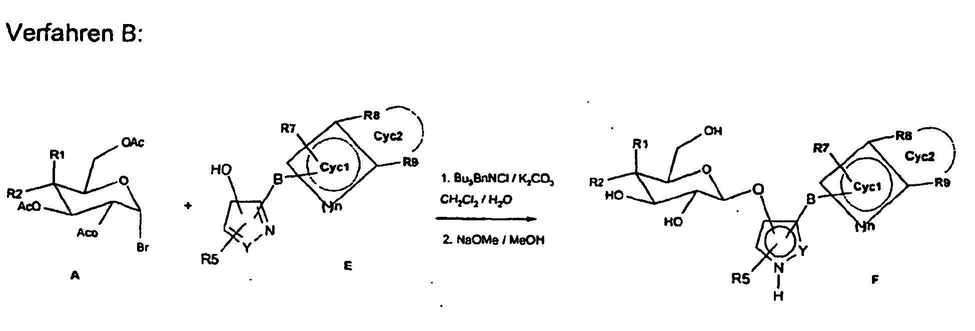

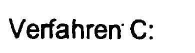

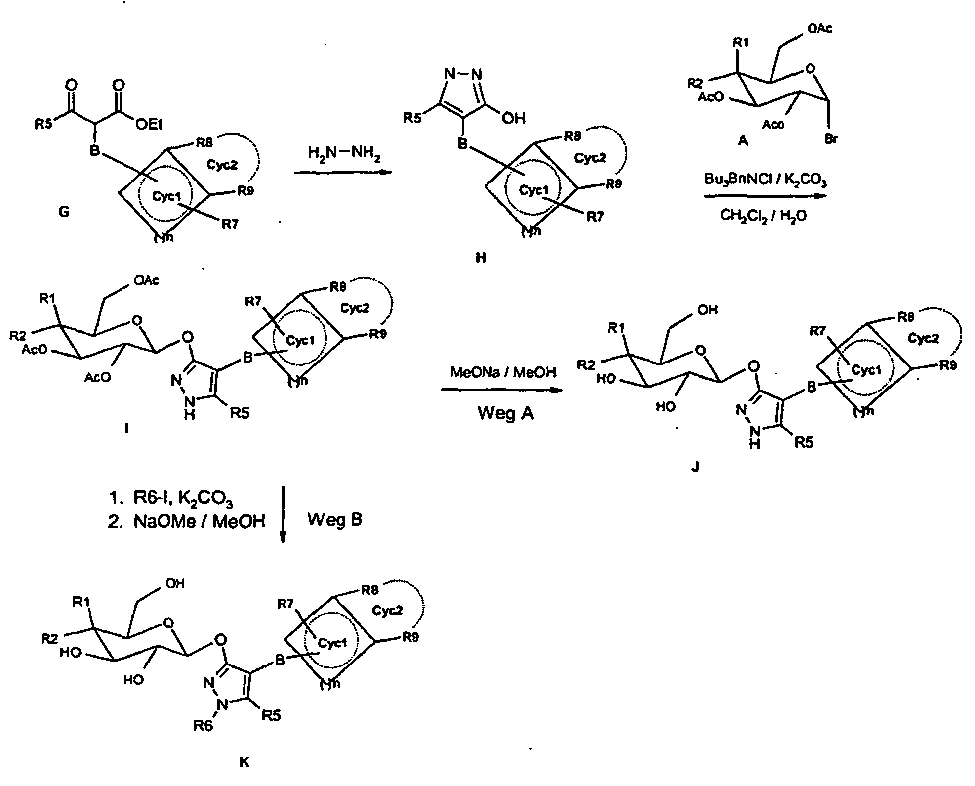

Die dargestellten Schemata der Verfahren A, B und C sprechen für sich selbst und sind für den Fachmann so ausführbar. Nähere Einzelheiten sind dennoch im Experimentellen Teil angegeben. Gemäß den Verfahren A, B und C wurden die Verbindungen der Beispiele 1 bis 31 erhalten. Andere Verbindungen der Formel I können entsprechend oder nach bekannten Verfahren erhalten werden.

Die Verbindung(en) der Formel I können auch in Kombination mit weiteren Wirkstoffen verabreicht werden.

Als weitere Wirkstoffe für die Kombinationspräparate sind geeignet:

Alle Antidiabetika, die in der Roten Liste 2001, Kapitel 12 genannt sind. Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.
Antidiabetika umfassen Insulin und Insulinderivate, wie z.B. Lantus® (siehe www.lantus.com) oder HMR 1964, schnell wirkende Insuline (siehe US 6,221,633), GLP-1-Derivate wie z.B. diejenigen die in WO 98/08871 von Novo Nordisk A/S offenbart wurden, sowie oral wirksame hypoglykämische Wirkstoffe.
Die oral wirksamen hypoglykämischen Wirkstoffe umfassen vorzugsweise Sulphonylfharnstoffe, Biguanidine, Meglitinide, Oxadiazolidindione, Thiazolidindione, Glukosidase-Inhibitoren, Glukagon-Antagonisten, GLP-1-Agonisten,
Kaliumkanalöffner, wie z.B. diejenigen, die in WO 97/26265 und WO 99/03861 von Novo Nordisk A/S offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, den Fettstoffwechsel verändernde Verbindungen wie antihyperlipidämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Kaliumkanal der Betazellen wirken.

Bei einer Ausführungsform der Erfindung werden die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin verabreicht.

Bei einer Ausführungsform der Erfindung werden die Verbindungen der Formel I in Kombination mit einem Cholesterinresorptionsinhibitor, wie z.B. Ezetimibe, Tiqueside, Pamaqueside, verabreicht.

Bei einer Ausführungsform der Erfindung werden die Verbindungen der Formel I in Kombination mit einem PPAR gamma Agonist, wie z.B. Rosiglitazon, Pioglitazon, JTT-501, Gl 262570, verabreicht.

Bei einer Ausführungsform der Erfindung werden die Verbindungen der Formel I in Kombination mit PPAR alpha Agonist, wie z.B. GW 9578, GW 7647, verabreicht.

Bei einer Ausführungsform der Erfindung werden die Verbindungen der Formel I in Kombination mit einem gemischten PPAR alpha/gamma Agonisten, wie z.B. GW 1536, AVE 8042, AVE 8134, AVE 0847, AVE 0897 oder wie in WO 00/64888, WO 00/64876, WO 03/20269 beschrieben verabreicht.

Bei einer Ausführungsform der Erfindung werden die Verbindungen der Formel I in Kombination mit einem Fibrat, wie z.B. Fenofibrat, Clofibrat, Bezafibrat, verabreicht.

Bei einer Ausführungsform der Erfindung werden die Verbindungen der Formel I in Kombination mit einem MTP-Inhibitor, wie z.B. Implitapide , BMS-201038, R-103757, verabreicht.

Bei einer Ausführungsform der Erfindung werden die Verbindungen der Formel I in Kombination mit Gallensäureresorptionsinhibitor (siehe z.B. US 6,245,744 oder US 6,221,897), wie z.B. HMR 1741, verabreicht.

Bei einer Ausführungsform der Erfindung werden die Verbindungen der Formel I in Kombination mit einem CETP-Inhibitor, wie z.B. JTT-705 , verabreicht.

Bei einer Ausführungsform der Erfindung werden die Verbindungen der Formel I in Kombination mit einem polymeren Gallensäureadsorber, wie z.B. Cholestyramin, Colesevelam, verabreicht.

Bei einer Ausführungsform der Erfindung werden die Verbindungen der Formel I in Kombination mit einem LDL-Rezeptorinducer (siehe US 6,342,512), wie z.B. HMR1171, HMR1586, verabreicht.

Bei einer Ausführungsform der Erfindung werden die Verbindungen der Formel I in Kombination mit einem ACAT-Inhibitor, wie z.B. Avasimibe, verabreicht.

Bei einer Ausführungsform der Erfindung werden die Verbindungen der Formel I in Kombination mit einem Antioxidans, wie z.B. OPC-14117, verabreicht.

Bei einer Ausführungsform der Erfindung werden die Verbindungen der Formel I in Kombination mit einem Lipoprotein-Lipase Inhibitor, wie z.B. NO-1886, verabreicht.

Bei einer Ausführungsform der Erfindung werden die Verbindungen der Formel I in Kombination mit einem ATP-Citrat-Lyase Inhibitor, wie z.B. SB-204990, verabreicht.

Bei einer Ausführungsform der Erfindung werden die Verbindungen der Formel I in Kombination mit einem Squalen Synthetase Inhibitor, wie z.B. BMS-188494, verabreicht.

Bei einer Ausführungsform der Erfindung werden die Verbindungen der Formel I in Kombination mit einem Lipoprotein(a) antagonist, wie z.B. CI-1027 oder Nicotinsäure, verabreicht.

Bei einer Ausführungsform der Erfindung werden die Verbindungen der Formel I in Kombination mit einem Lipase Inhibitor, wie z.B. Orlistat, verabreicht.

Bei einer Ausführungsform der Erfindung werden die Verbindungen der Formel I in Kombination mit Insulin verabreicht.
Bei einer Ausführungsform werden die Verbindungen der Formel I in Kombination mit einem Sulphonylhamstoff, wie z.B. Tolbutamid, Glibenclamid, Glipizid oder Glimepirid verabreicht.
Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.
Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]-phenyl]methyl]-2,4-thiazolidindion, verabreicht.
Bei einer Ausführungsform werden die Verbindungen der Formel I in Kombination mit einem *α*-Glukosidase-Inhibitor, wie z.B. Miglitol oder Acarbose, verabreicht.
Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.
Bei einer Ausführungsform werden die Verbindungen der Formel I in Kombination mit mehr als einer der vorstehend genannten Verbindungen, z.B. in Kombination mit einem Sulphonylhamstoff und Metformin, einem Sulphonylhamstoff und Acarbose, Repaglinid und Metformin, Insulin und einem Sulphonylharnstoff, Insulin und Metformin, Insulin und Troglitazon, Insulin und Lovastatin, etc. verabreicht.

Bei einer weiteren Ausführungsform werden die Verbindungen der Formel I in Kombination mit CART-Modulatoren (siehe "Cocaine-amphetamine-regulated transcript influences energy metabolism, anxiety and gastric emptying in mice" Asakawa, A, et al., M.:Hormone and Metabolic Research (2001), 33(9), 554-558), NPY-Antagonisten z.B. Naphthalin-1-sulfonsäure {4-[(4-amino-quinazolin-2-ylamino)-methyl]-cyclohexylmethyl}- amid; hydrochlorid (CGP 71683A)), MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)), Orexin-Antagonisten (z.B. 1-(2-Methyl-benzoxazol-6-yl)-3-[1,5]naphthyridin-4-yl-harnstoff; hydrochloride (SB-334867-A)), H3-Agonisten (3-Cyclohexyl-1-(4,4-dimethyl-1,4,6,7-tetrahydro-imidazo[4,5-c]pyridin-5-yl)-propan-1- on Oxalsäuresalz (WO 00/63208)); TNF-Agonisten, CRF-Antagonisten (z.B. [2-Methyl-9-(2,4,6-trimethyl-phenyl)-9H-1,3,9-triaza-fluoren-4-yl]-dipropyl-amin (WO 00/66585)), CRF BP-Antagonisten (z.B. Urocortin), Urocortin-Agonisten, *β*3-Agönisten (z.B. 1-(4-Chloro-3-methanesulfonylmethyl-phenyl)-2-[2-(2,3-dimethyl-1H-indol-6-yloxy)-ethylamino]-ethanol; hydrochloride (WO 01/83451)), MSH (Melanocyt-stimulierendes Hormon)-Agonisten, CCK-A Agonisten (z.B. {2-[4-(4-Chloro-2,5-dimethoxy-phenyl)-5-(2-cyclohexyl-ethyl)-thiazol-2-ylcarbamoyl]-5,7- dimethyl-indol-1-yl}-acetic acid Trifluoressigsäuresalz (WO 99/15525)); Serotonin-Wiederaufnahme-Inhibitoren (z.B. Dexfenfluramine), gemischte Sertonin- und noradrenerge Verbindungen (z.B. WO 00/71549), 5HT-Agonisten z.B. 1-(3-Ethyl-benzofuran-7-yl)-piperazin Oxalsäuresalz (WO 01/09111), Bombesin-Agonisten, Galanin-Antagonisten, Wachstumshormon (z.B. humanes Wachstumshormon), Wachstumshormon freisetzende Verbindungen (6-Benzyloxy-1-(2-diisopropylamino-ethylcarbamoyl)-3,4-dihydro-1H-isoquinoline-2-carboxylic acid tert-butyl ester (WO 01/85695)), TRH-Agonisten (siehe z.B. EP 0 462 884) entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881),
DA-Agonisten (Bromocriptin, Doprexin), Lipase/Amylase-Inhibitoren (z.B. WO 00/40569), PPAR-Modulatoren (z.B. WO 00/78312), RXR-Modulatoren oder TR-*β-*Agonisten verabreicht.

Bei einer Ausführungsform der Erfindung ist der weitere Wirkstoff Leptin; siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform ist der weitere Wirkstoff Dexamphatamin oder Amphetamin.
Bei einer Ausführungsform ist der weitere Wirkstoff Fenfluramin oder Dexfenfluramin.
Bei noch einer Ausführungsform ist der weitere Wirkstoff Sibutramin.
Bei einer Ausführungsform ist der weitere Wirkstoff Orlistat.
Bei einer Ausführungsform ist der weitere Wirkstoff Mazindol oder Phentermin.
Bei einer Ausführungsform werden die Verbindungen der Formel I in Kombination mit Ballaststoffen, vorzugsweise unlöslichen Ballaststoffen (siehe z.B. Carob/ Caromax® (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties &Food Ingredients GmbH, Industriepark Höchst, 65926 Frankfurt / Main)) verabreicht. Die Kombination mit Caromax® kann in einer Zubereitung erfolgen, oder durch getrennte Gabe von Verbindungen der Formel I und Caromax®. Caromax® kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

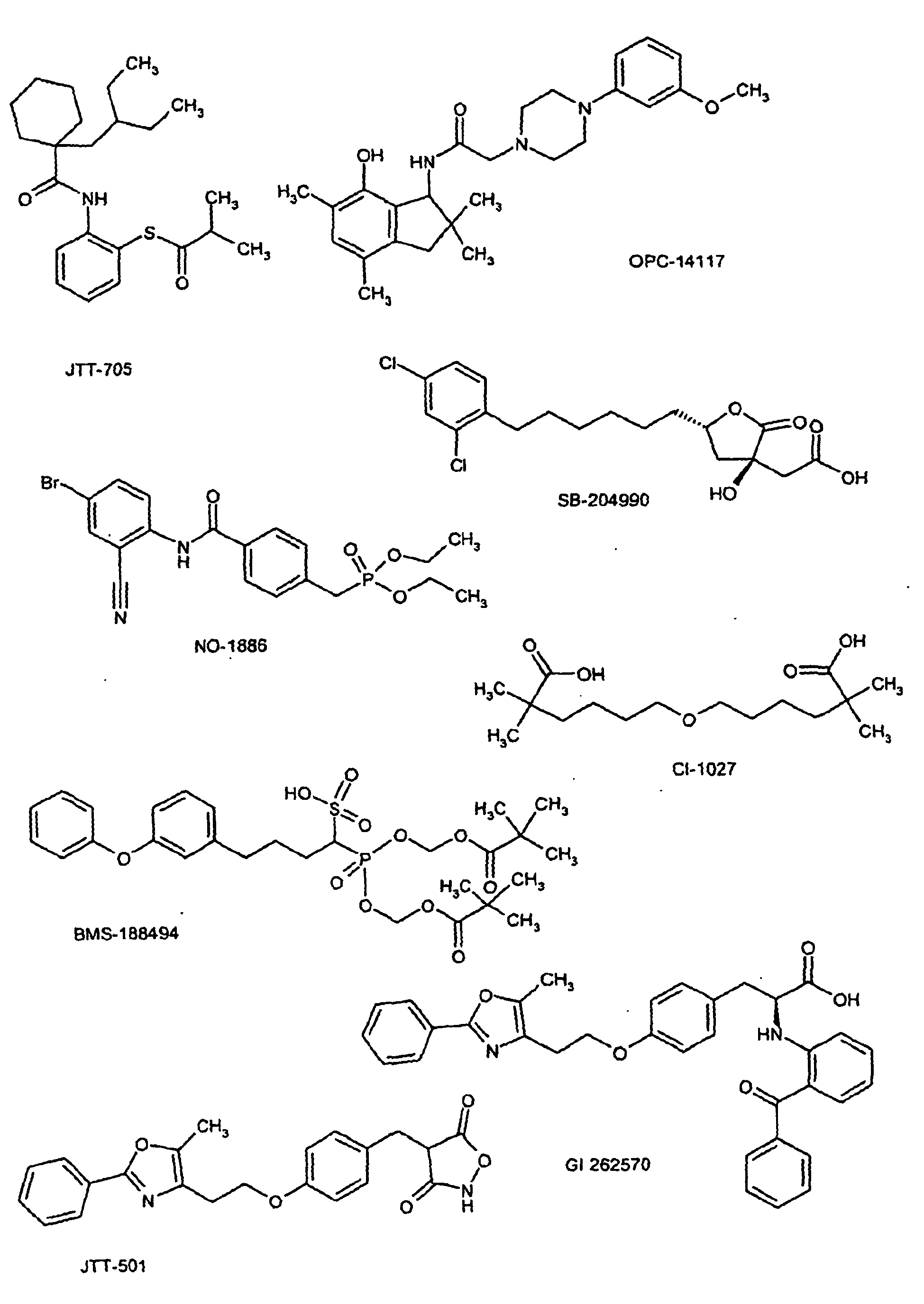

Die nachfolgend aufgeführten Beispiele dienen zur Erläuterung der Erfindung, ohne diese jedoch einzuschränken.

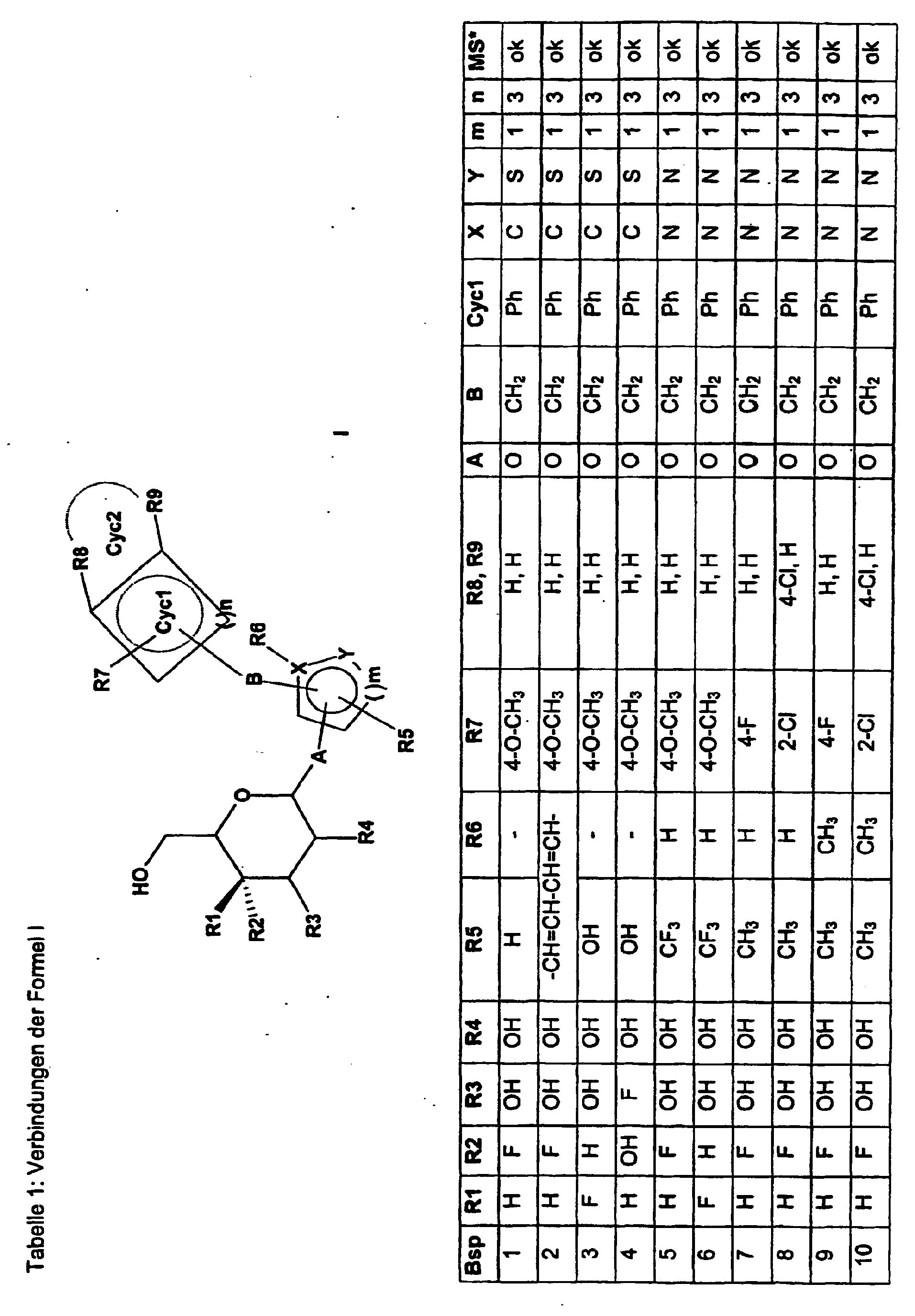

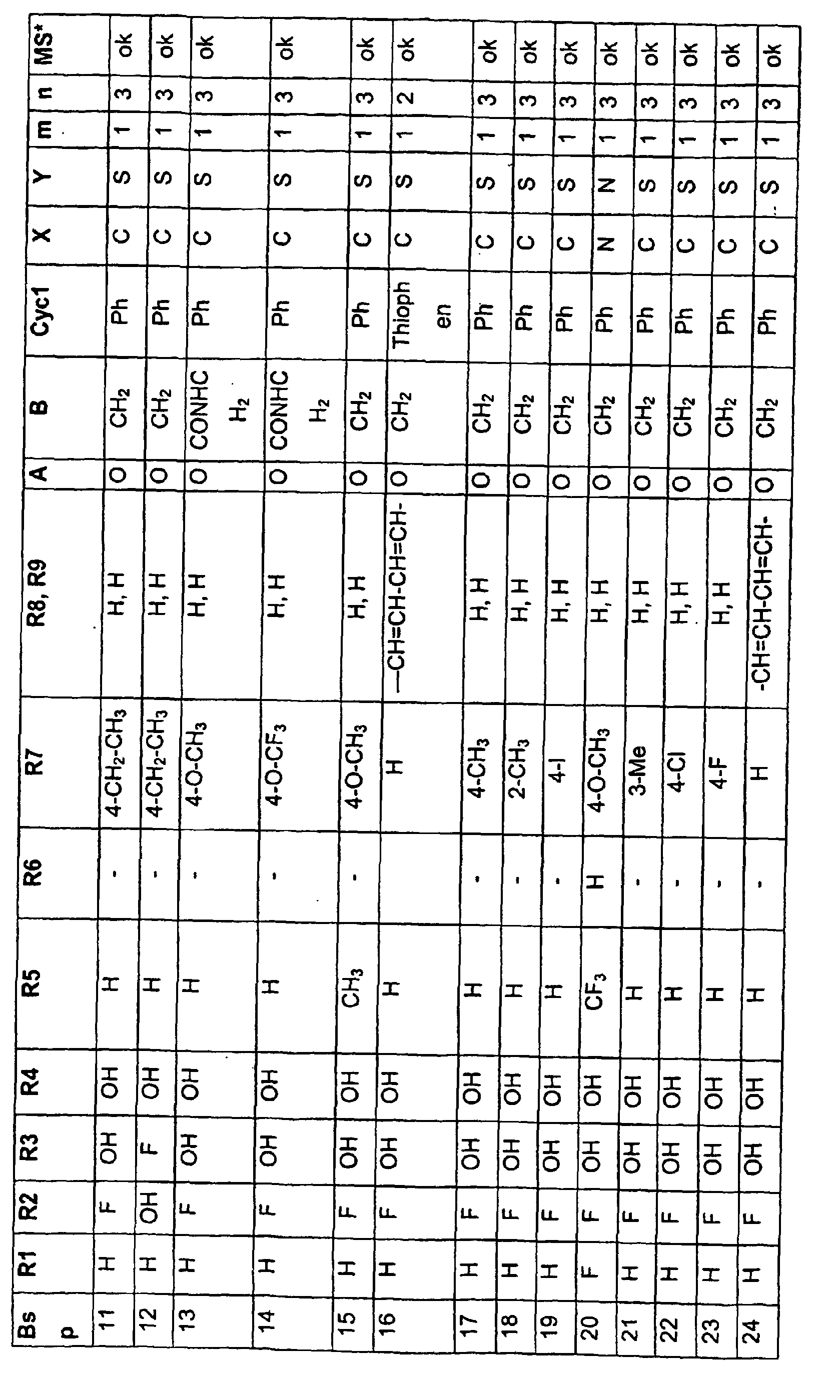

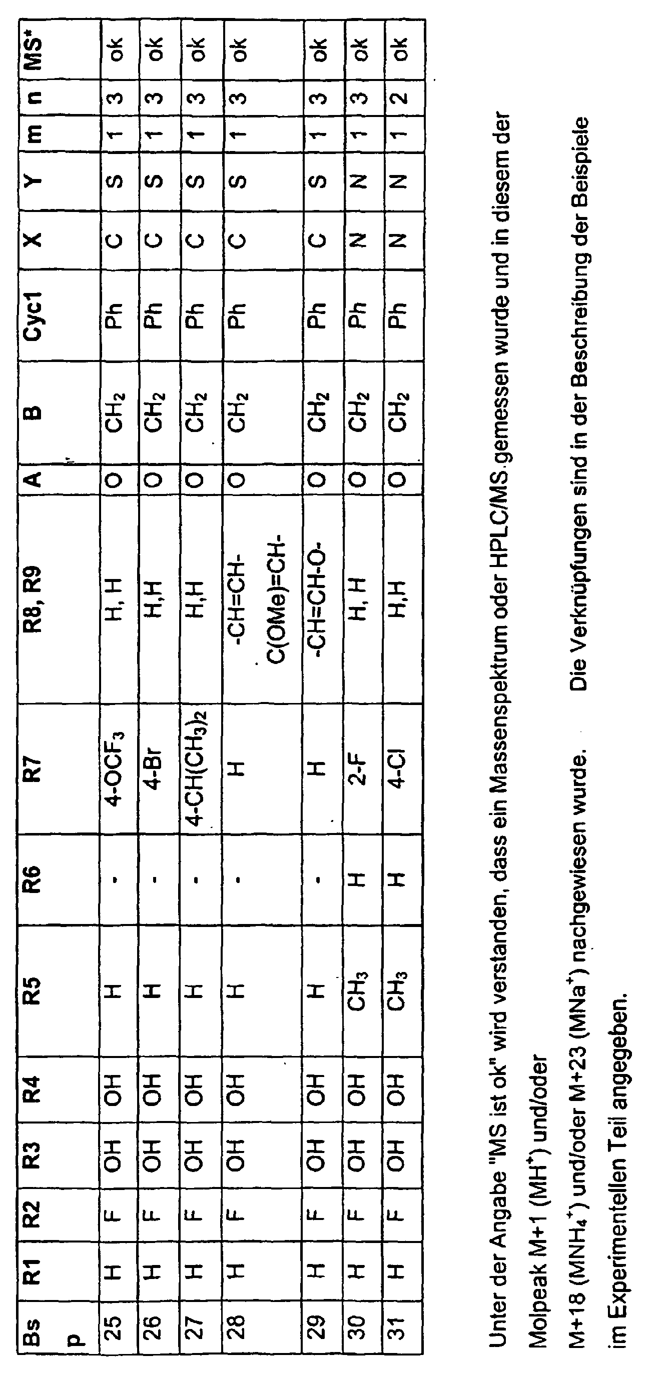

Die Verbindungen der Formel I zeichnen sich durch günstige Wirkungen auf den Glucosestoffwechsel aus, sie senken insbesondere den Blutzuckerspiegel und sind zur Behandlung von Typ 1 und Typ 2 Diabetes geeignet. Die Verbindungen können daher allein oder in Kombination mit weiteren Blutzucker-senkenden Wirkstoffen (Antidiabetika) eingesetzt werden.
Die Verbindungen der Formel I eignen sich weiterhin zur Prävention und Behandlung von Diabetischen Spätschäden, wie z.B. Nephropatie, Retinopathie, Neuropathie sowie Syndrom X, Obesitas, Herzinfarkt, Myocardialem Infarkt, peripheren arteriellen Verschlusskrankheiten, Thrombosen, Arteriosklerose, Entzündungen, Immunkrankheiten, Autoimmunkrankheiten, wie z.B. AIDS, Asthma, Osteoporose, Krebs, Psoriasis, Alzheimer, Schizophrenie und Infektionskrankheiten, bevorzugt ist die Behandlung von Typ 1 und Typ 2 Diabetes sowie zur Prävention und Behandlung von Diabetischen Spätschäden, Syndrom X und Obesitas.

Die Wirksamkeit der Verbindungen wurde wie folgt getestet:

Präparation von Bürstensaummembran-Vesikeln aus dem Dünndarm von Kaninchen, Ratte und Schwein

Die Präparation von Bürstensaummembran-Vesikeln aus den Darmzellen des Dünndarms erfolgte mit der sog. Mg²⁺-Präzipitationsmethode. Die Mucosa aus dem Dünndarm wurde abgeschabt und in 60 ml eiskaltem Tris/HCl-Puffer (ph 7,1) / 300 mM Mannit, 5 mM EGTA suspendiert. Nach dem Verdünnen auf 300 ml mit eiskaltem destilliertem Wasser wurde mit einem Ultraturrax (18-Stab, IKA Werk Staufen, BRD) 2 x 1 Minute bei 75 % max. Leistung unter Eiskühlung homogenisiert. Nach Zugabe von 3 ml 1 M MgCl₂-Lösung (Endkonzentration 10 mM) lässt man exakt 15 Minuten bei 0° C stehen. Durch die Zugabe von Mg²⁺ aggregieren die Zell-membranen und präzipitieren, mit Ausnahme der Bürstensaummembranen. Nach einer 15-minütigen Zentrifugation bei 3 000 x g (5 000 rpm, SS-34-Rotor) wird der Niederschlag verworfen und der Überstand, der die Bürstensaummembranen enthält, 30 Minuten bei 26 700 x g (15 000 rpm, SS-34-Rotor) zentrifugiert. Der Überstand wird verworfen, der Niederschlag in 60 ml 12 mM Tris/HCl-Puffer (ph 7,1) / 60 mM Mannit, 5 mM EGTA mit einem Potter Elvejhem Homogenisator (Braun, Melsungen, 900 rpm, 10 Hübe) rehomogenisiert. Nach Zugabe von 0,1 ml 1 M MgCl₂-Lösung und 15-minütiger Inkubation bei 0°C wird erneut 15 Minuten bei 3 000 x g zentrifugiert. Der Überstand wird anschließend nochmals 30 Minuten bei 46 000 x g (20 000 rpm, SS-34-Rotor) zentrifugiert. Der Niederschlag wird in 30 ml 20 mM Tris/Hepes-Puffer (pH 7,4) / 280 mM Mannit aufgenommen und durch 20 Hübe in einem Potter Elveihem Homogenisator bei 1 000 rpm homogen resuspendiert. Nach 30-minütiger Zentrifugation bei 48 000 x g (20 000 rpm, SS-34-Rotor) wurde der Niederschlag in 0,5 bis 2 ml Tris/Hepes-Puffer (pH 7,4) / 280 mM Mannit (Endkonzentration 20 mg/ml) aufgenommen und mit Hilfe einer Tuberkulinspritze mit einer 27 Gauge-Nadel resuspendiert.
Die Vesikel wurden entweder unmittelbar nach der Präparation für Markierungs- oder Transportuntersuchungen verwendet oder wurden bei -196°C in 4 mg Portionen in flüssigem Stickstoff aufbewahrt.
Für die Präparation von Bürstensaummembranvesikeln aus Rattendünndarm wurden 6 bis 10 männliche Wistar-Ratten (Tierzucht Kastengrund, Aventis Pharma), durch zervikale Dislokation getötet, die Dünndärme entnommen und mit kalter isotonischer Kochsalzlösung gespült. Die Därme wurden aufgeschnitten und die Mucosa abgeschabt. Die Aufarbeitung zur Isolierung von Bürstensaummembranen erfolgte wie oben beschrieben. Zur Abtrennung von Cytoskelettanteilen wurden die Bürstensaummembranvesikel aus Rattendünndarm mit KSCN als chaotropem Ion behandelt.

Für die Präparation von Bürstensaummembranen aus Kaninchendünndarm wurden Kaninchen durch intravenöse Injektion von 0,5 ml einer wässrigen Lösung von 2,5 mg Tetracain-HCl, 100 mg m-Butramid und 25 mg Mebezoniumjodid getötet. Die Dünndärme wurden entnommen, mit eiskalter physiologischer Kochsalzlösung gespült und in Kunststoffbeutel unter Stickstoff bei - 80 °C eingefroren und 4 bis 12 Wochen gelagert. Zur Präparation der Membranvesikel wurden die eingefrorenen Därme bei 30°C im Wasserbad aufgetaut und anschließend die Mucosa abgeschabt. Die Aufarbeitung zu Membranvesikeln erfolgte wie oben beschrieben.

Zur Präparation von Bürstennsaummembranvesikeln aus Schweinedarm wurden Jejunumsegmente eines frisch geschlachteten Schweines mit eiskalter isotonischer Kochsalzlösung gespült und in Plastikbeuteln unter Stickstoff bei - 80°C eingefroren. Die Präparation der Membranvesikel erfolgte wie oben beschrieben.

### Präparation von Bürstensaummembranvesikeln aus dem Nierenkortex der Rattenniere

Die Präparation der Bürstensaummembranvesikel aus dem Kortex der Rattenniere erfolgte nach der Methode von Biber et al. Die Nieren von 6 bis 8 Ratten (200 bis 250 g) wurden entnommen und von jeder Niere wurde der Kortex als ca. 1 mm starke Schicht abgetragen. Die Nieren wurden in 30 ml eiskaltem 12 mM Tris/HC1-Puffer (pH 7,4) / 300mM Mannit aufgenommen und unter Eiskühlung 4 x 30 Sekunden mit einem Ultraturraxstab (Stufe 180 V) homogenisiert. Nach der Zugabe von 42 ml eiskaltem destilliertem Wasser wurden 850 µl einer 1 M MgCl₂-Lösung zugegeben. Nach 15-minütiger Inkubation bei 0°C wurde 15 Minuten bei 4 500 rpm (Sorvall SS-34-Rotor) zentrifugiert. Der Niederschlag wurde verworfen, der Überstand 30 Minuten bei 16 000 rpm zentrifugiert. Nach Resuspendierung des Niederschlags in 60 ml 6 mM Tris/HCl-Puffer (pH 7,4) /150 mM Mannit / 2,5 mM EGTA durch 10 Hübe in einem Potter-Elvejhem Homogenisator (900 rpm) wurde nach Zugabe von 720 µl 1 mM MgCl₂-Lösung 15 Minuten bei 0°C inkubiert. Nach 15-minütiger Zentrifugation bei 4 500 rpm (SS-34-Rotor) wurde der resultierende Überstand 30 Minuten bei 16000 rpm zentrifugiert. Der Überstand wurde durch 10 Hübe in 60 ml 20 mM Tris/Hepes-Puffer (pH 7,4) / 280 mM Mannit homogenisiert und die entstehende Suspension anschließend 30 Minuten bei 20 000 rpm zentrifugiert. Der Niederschlag wurde mit Hilfe einer Tuberkulinspritze mit einer 27 Gauge-Nadel in 20 mM Tris/HCl-Puffer (pH 7,4) / 280 mM Mannit resuspendiert und auf eine Proteinkonzentration von 20 mg/ml eingestellt.

Messung der Glukoseaufnahme durch Bürstensaummembranvesikel

Die Aufnahme von [¹⁴C]-markierter Glukose in Bürstensaummembranvesikel wurde mittels der Membranfiltrationsmethode gemessen. 10 µl der Bürstensaummembranvesikelsuspension in 10 mM Tris/Hepes-Puffer (pH 7.4)/300 mM Mannitol wurden bei 30°C zu 90 µl einer Lösung von 10 *µ*M [¹⁴C]D-Glukose und den entsprechenden Konzentrationen der betreffenden Hemmstoffe (5-200 µM) in 10 mM Tris/Hepes-Puffer (pH 7.4)/ 100 mM NaCl/100 mM Mannitol gegeben.
Nach 15 sec. Inkubation wurde der Transportprozess durch Zugabe von 1 ml eiskalter Stopplösung (10 mM Tris/Hepes-Puffer (pH 7.4)/ 150 mM KCl) angehalten und die Vesikelsuspension wurde sofort bei einem Vakuum von 25 bis 35 mbar über ein Membranfilter aus Cellulosenitrat (0,45 µm, 25 mm, Durchmesser, Schleicher & Schüll) abgesaugt. Der Filter wurde mit 5 ml eiskalter Stopplösung nachgewaschen.
Jeder Messpunkt wurde als Doppel- oder Dreifachbestimmung ausgeführt.
Zur Messung der Aufnahme radioaktiv markierter Substrate wurde der Membranfilter in 4 ml eines entsprechenden Szintillators (Quickszint 361, Zinsser Analytik GmbH, Frankfurt am Main) aufgelöst und die Radioaktivität durch Flüssigkeisszintillationsmessung bestimmt. Die gemessenen Werte wurden nach Eichung des Gerätes mit Hilfe von Standardproben und nach Korrektur evtl. vorhandener Chemilumiszenz als dpm (Disintegrations per minute) erhalten.

Der Aktivitätsvergleich der Wirkstoffe wird anhand von IC₅₀ Daten durchgeführt, die im Transport-Assay an Dünndarm- Bürstensaummembranvesikeln des Kaninchens für ausgewählte Substanzen erhalten wurden. (Die Absolutwerte können Spezies- und Versuchs-abhängig sein)

| Beispiel Nr. | IC50 [µM] |
|---|---|
| Phlorizin | 16 |
| 1 | 4 |
| 2 | 0.4 |
| 3 | 0.3 |

Nachfolgend wird die Herstellung verschiedener Beispiele detailliert beschrieben, die übrigen Verbindungen der Formel I wurden analog erhalten:

### Experimenteller Teil:

### Reaktions-Schema: Synthese von α-Bromglykosiden

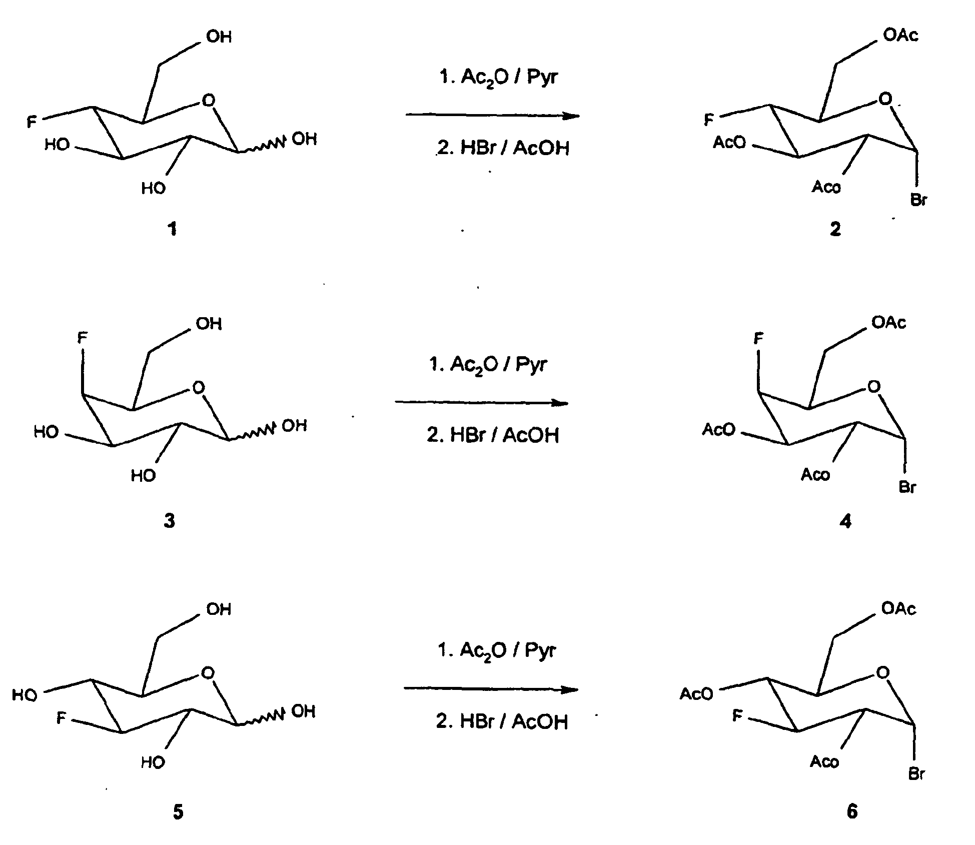

### 1-Bromo-4-deoxy-4-fluoro-2,3,6-tri-O-acetyl-alpha-D-glucose (2)

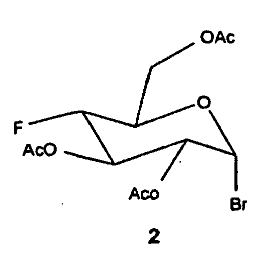

5,0 g (27.5 mmol) 4-Deoxy-4-fluoro-D-glucopyranose 1 (Apollo) werden in 50 ml Pyridin und 50 ml Essigsäureanhydrid suspendiert. Die Reaktionslösung wird 4 Stunden bei 45°C gerührt. Dabei erhält man eine klare Reaktionslösung, die eingeengt wird. Man erhält 12,0 g Rohprodukt. Dieses Rohprodukt wird in 160 ml 33%-iger HBr in Eisessig gelöst und 2 Stunden bei Raumtemperatur stehen gelassen. Die Reaktionslösung wird dann auf eine Mischung von 300 g Eis und 300 ml Ethylacetat gegossen. Die organische Phase wird zweimal mit wässriger NaCl-Lösung gewaschen, über wenig Kieselgel filtriert und eingeengt. Der Rückstand wird durch Chromatographie an Kieselgel (Ethylacetat/Heptan = 1/1) getrennt. Man erhält 8.19 g (80 % über 2 Stufen) 2 als hellgelben Feststoff.

### 1-Bromo-4-deoxy-4-fluoro-2,3,6 Tri-O-acetyl-alpha-D-galactose (4)

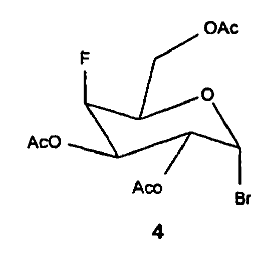

100 mg (0.55 mmol) 3 mit 3.5 ml Pyridin und 3.5 ml Essigsäureanhydrid werden analog der Herstellung von Verbindung 2 umgesetzt. Man erhält 89 mg (44 %) 4 als amorphen Feststoff.

### 1-Bromo-3-deoxy-3-fluoro-2,4,6-tri-O-acetyl-alpha-D-glucose (6)

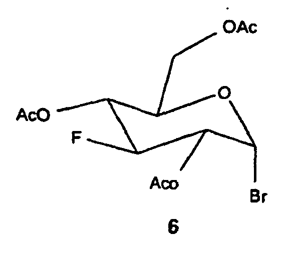

335 mg (1.84 mmol) 5 mit 10 ml Pyridin und 10 ml Essigsäureanhydrid werden analog der Herstellung von Verbindung 2 umgesetzt. Man erhält 628 mg (92 %) 6 als amorphen Feststoff.

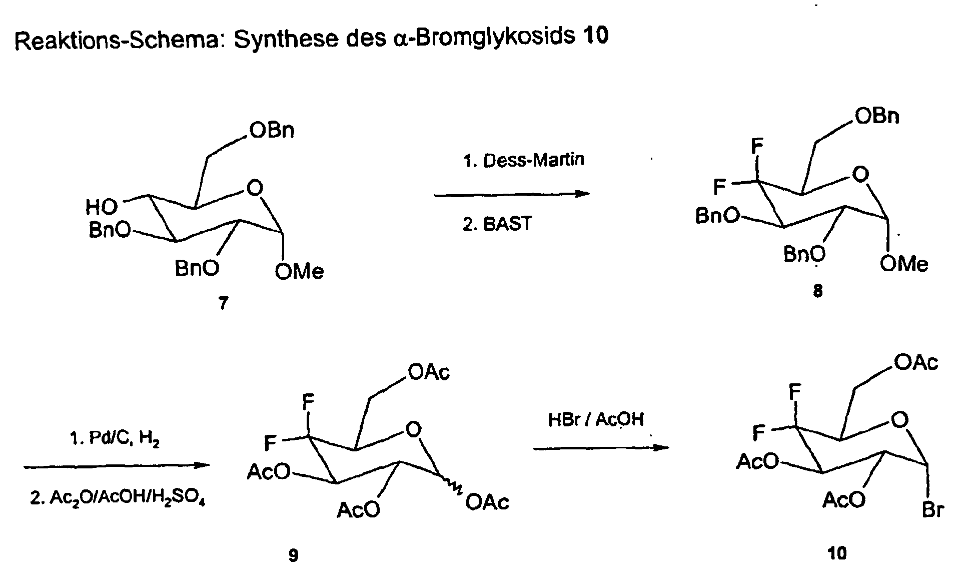

### 1-Methoxy-4-deoxy-4,4-difluoro-2,3,6-tri-O-benzyl-alpha-D-glucose (8)

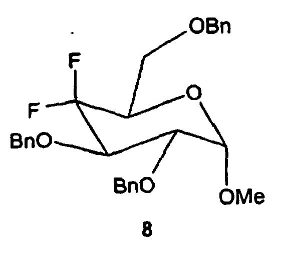

3.69 g (7.9 mmol) 1-Methoxy-2,3,6-tri-O-benzyl-alpha-D-glucose 7 (Tetrahedron Asymmetry 2000, 11, 385-387) werden in 110 ml Methylenchlorid gelöst, und unter einer Argonatmosphere werden 3.6 g (8.5 mmol) Dess-Martin-Reagenz (Aldrich) zugetropft. Nach 3 Stunden bei Raumtemperatur wird mit 300 ml Essigester / n-Heptan (1:1) verdünnt und 1 x mit NaHCO₃ und 1 x mit Na₂S₂O₃-Lösung gewaschen. Die organische Phase wird über Kieselgel filtriert und eingeengt. Der Rückstand wird durch Chromatographie an Kieselgel (Ethylacetat / n-Heptan 1:1) getrennt. Man erhält 2,90 g (79 %) des Ketons. Dieses wird in 30 ml Methylenchlorid gelöst und unter einer Argonatmosphere werden 4.0 ml BAST ([Bis(2-methoxyethyl)amino]schwefeltrifluorid, Aldrich) zugetroft. Nach 20 Stunden bei Raumtemperatur wird mit 200 ml Essigester verdünnt und mit kalter NaHCO₃-Lösung vorsichtig (sprudelt stark) gewaschen. Die organische Phase wird über Kieselgel filtriert und eingeengt. Der Rückstand wird durch Chromatographie an Kieselgel (Ethylacetat/n-Heptan 1:1) getrennt. Man erhält 2.6 g (85 %) 8 als farbloses Öl.

### 4-Deoxy-4,4-difluoro-1,2,3,6-tetra-O-acetyl-alpha-D-glucose (9)

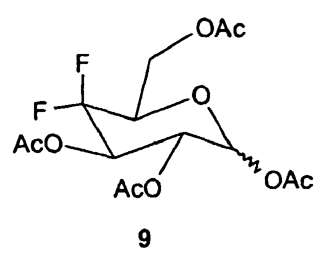

2.30 g (4.7 mmol) 8 und 2.0 g Pd/C (10 % Pd) werden in 150 ml Methanol und 10 ml Essigsäure gelöst und unter einer 5 bar Wasserstoffatmosphäre 16 h bei Raumtemperatur hydriert. Die Reaktionslösung wird eingeengt und der Rückstand mit Flashchromatographie (Methylenchlorid/Methanol/conz.Amoniak, 30/5/1) gereinigt. Ausbeute 850 mg (83%) 1-Methoxy-4-deoxy-4,4-difluoro-alpha-D-glucose als weißer, amorpher Feststoff. C₇H₁₂F₂O₅ (214.17) MS(DCI): 215.4 (M + H⁺).
Davon werden 700 mg (3.3 mmol) in 3.5 ml Essigsäure und 6.3 ml Essigsäureandydrid gelöst. Nach Zugabe von 0.2 ml konz. H₂SO₄ wird 5 h bei 60 °C gerührt.. Die Reaktionslösung wird dann auf eine Mischung von 30 g Eis und 30 ml Ethylacetat gegossen. Die organische Phase wird zweimal mit wässriger NaCl-Lösung gewaschen, über wenig Kieselgel filtriert und eingeengt. Der Rückstand wird durch Chromatographie an Kieselgel (Ethylacetat/n-Heptan 1:1) getrennt. Man erhält 300 mg (25 %) 9 als Anomerengemisch. C₁₄H₁₈F₂O₉ (368.29) MS(DCI): 369.3 (M+H⁺).

### 1-Bromo-4-deoxy-4,4-difluoro-2,3,6-tri-O-acetyl-alpha-D-glucose (10)

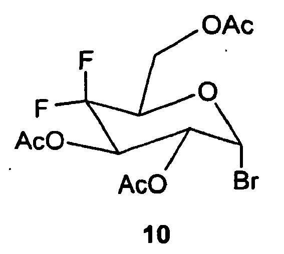

300 mg (0.8 mmol) Tetraacetat 9 werden in 13 ml 33 %iger HBr in Eisessig gelöst und 6 Stunden bei Raumtemperatur stehen gelassen. Die Reaktionslösung wird dann auf eine Mischung von 10 g Eis und 10 ml Ethylacetat gegossen. Die organische Phase wird zweimal mit wässriger NaCl-Lösung gewaschen, über wenig Kieselgel filtriert und eingeengt. Der Rückstand wird durch Chromatographie (SiO₂) (Ethylacetat/Heptan 1:1) getrennt. Man erhält 112 mg (35 %) 10 als farblosen Feststoff. C₁₂H₁₅BrF₂O₇ (389.15) MS(DCI): 389.2 (M+H⁺).

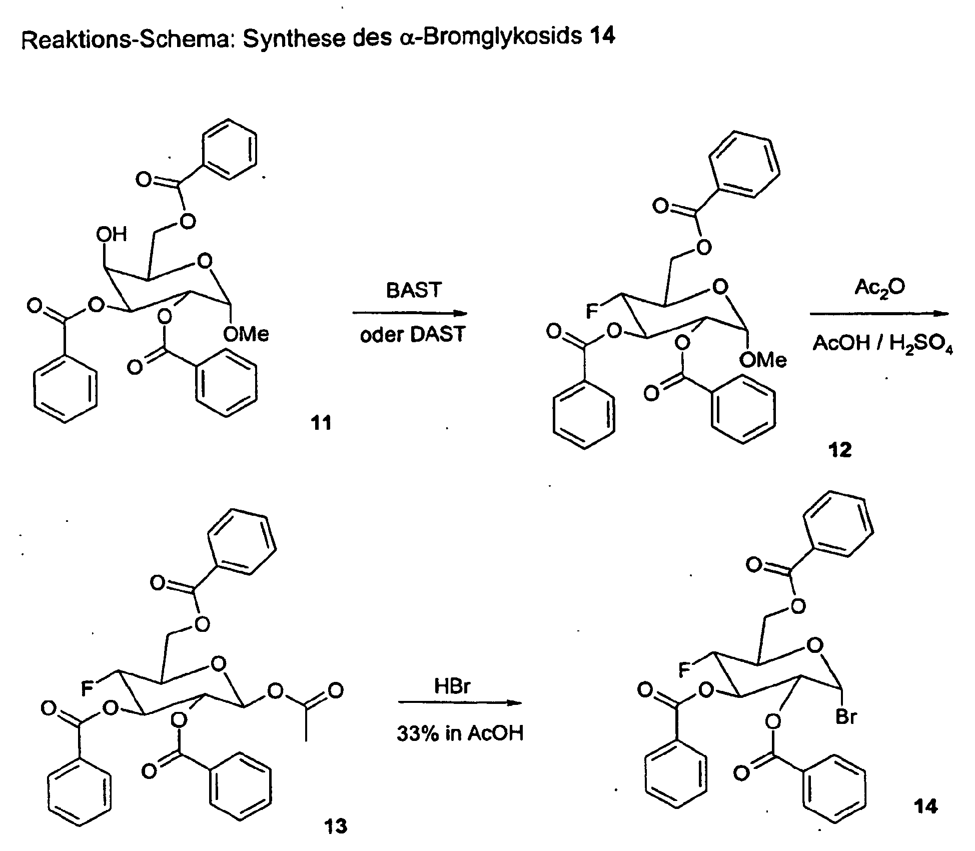

### Methyl-2,3,6-tri-O-benzoyl-4-fluoro-4-deoxy-α-D-glucopyranosid (12)

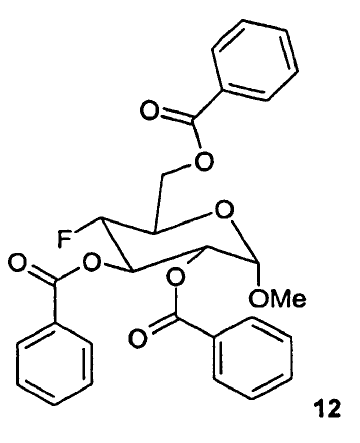

3,0 g Methyl 2,3,6-Tri-O-benzoyl-α-D-galactopyranosid (Reist et al., J.Org.Chem 1965, 30,2312) werden in Dichlormethan vorgelegt und auf -30°C gekühlt. Dann werden 3,06 ml [Bis(2-methoxyethyl)amino]sulfurtriflourid (BAST) zugetropft. Die Reaktionslösung wird auf Raumtemperatur erwärmt und 12 h gerührt. Der Ansatz wird mit Dichlormethan verdünnt und die organische Phase mit H₂O, NaHCO₃-Lsg. und gesättigter NaCl-Lsg. extrahiert. Die organische Phase wird über Na₂SO₄ getrocknet und eingeengt. Das Rohprodukt wird aus Ethylacetat und Heptan kristallisiert. Man erhält 1,95g des Produkts 12 als farblosen Feststoff. C₂₈H₂₅FO₈ (508.51) MS (ESI⁺) 526.18 (M+NH₄⁺). Alternativ kann die Reaktion auch unter Verwendung von 2,8 eq. Diethylaminosulfurtrifluorid (DAST) durchgeführt werden; hierbei wird die Reaktionslösung nach erfolgter Zugabe für 18 h refluxiert. Die Aufarbeitung erfolgt analog zu der oben beschriebenen.

### 1-O-Acetyl, 2,3,6-tri-O-benzoyl-4-fluoro-4-deoxy-glucose (13)

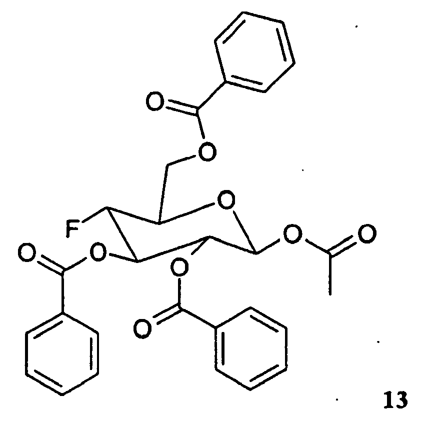

12,0 g Verbindung Methyl-2,3,6-tri-O-benzoyl-4-fluoro-4-deoxy-α-D-glucopyranosid werden in 150 ml Essigsäureanhydrid suspendiert. 8,4 ml konz. Schwefelsäure werden mit 150 ml Eisessig gemischt und unter Eiskühlung zum Ansatz gegeben. Der Ansatz rührt bei Raumtemperatur für 60 h. Das Reaktionsgemisch wird in NaHCO₃-Lsg. gegossen und diese Lösung mit Dichlormethan extrahiert. Die organische Phase wird mit NaCl-Lsg. gewaschen, mit Na₂SO₄ getrocknet und eingeengt. Der Rückstand wird aus Ethylacetat und Heptan umkristallisiert. Man erhält 5,97 g des Produkts 13 als farblosen Feststoff.
C₂₉H₂₅FO₉ (536.52) MS (ESI⁺) 554.15 (M+NH₄⁺).

### 1-Bromo-4-deoxy-4-fluoro-2,3,6-tri-O-benzoyl-alpha-D-glucose (14)

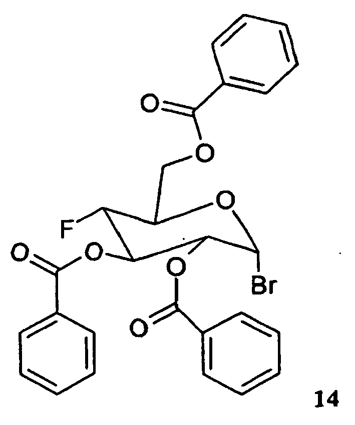

1,44 g 1-O-Acetyl,2,3,6-tri-O-benzoyl- 4-fluoro-4-deoxy-glucose werden in 20 ml Bromwasserstoffsäure in Eisessig (33%) gelöst und bei Raumtemperatur gerührt. Nach 5 Stunden wird der Ansatz auf Eiswasser gegeben, die wässrige Phase wird dreimal mit Dichlormethan extrahiert. Die gesammelte organische Phase wird mit gesättigter Natriumchloridlösung gewaschen, über Natriumsulfat getrocknet und zur Trockne eingeengt. Das Rohprodukt wird mit Ethylacetat/Heptan (70:30) über Kieselgel filtriert. Man erhält 1,40 g des Produkts 14 als farblosen Feststoff. C₂₇H₂₂BrFO₇ (557.37) MS (ESI⁺) 574.05/576.05 (M+NH₄⁺).

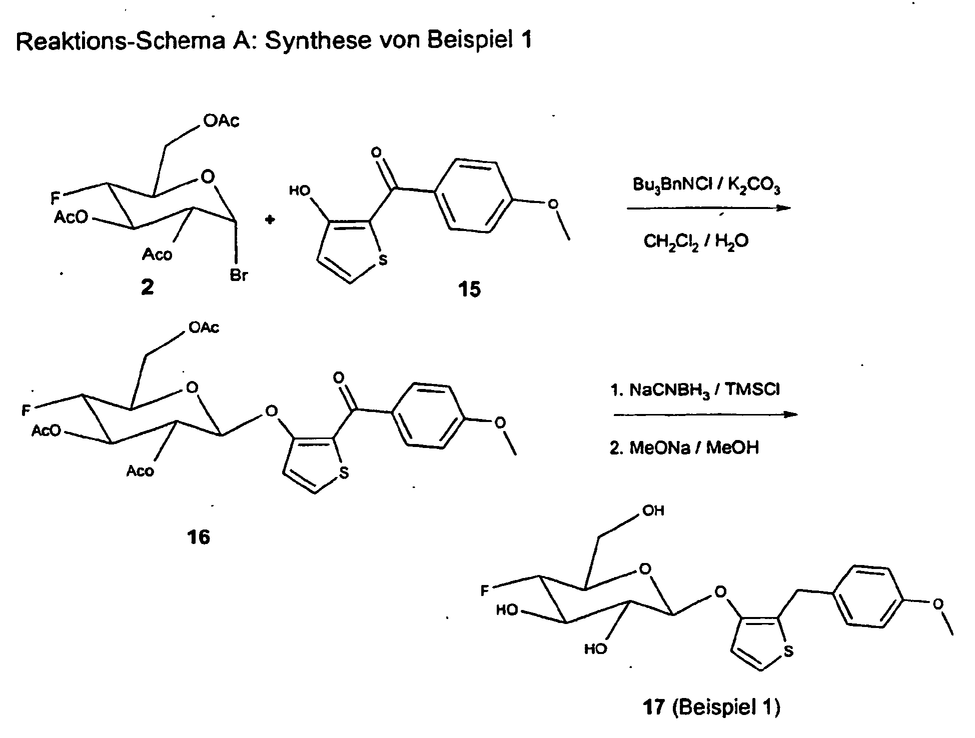

### Weitere Beispiel-Verbindungen:

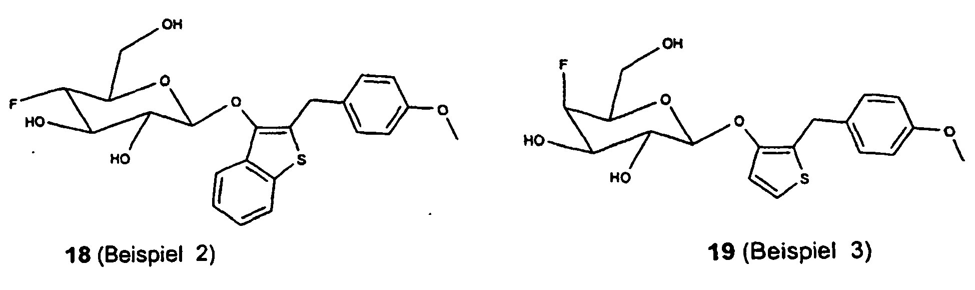

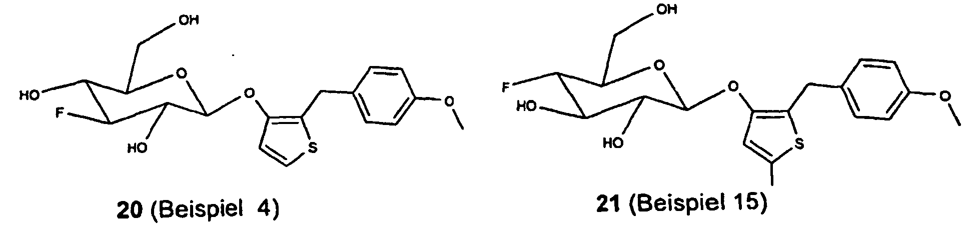

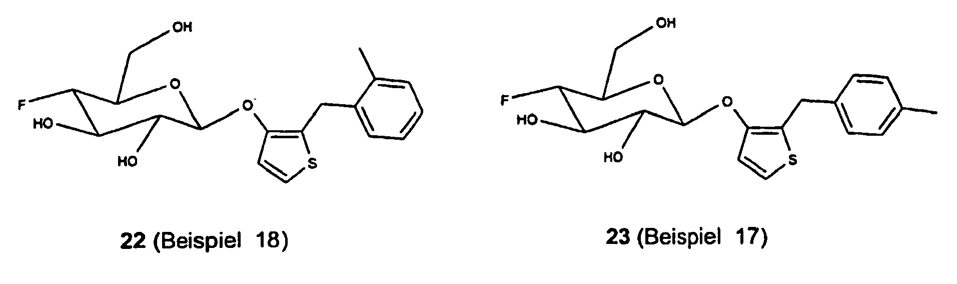

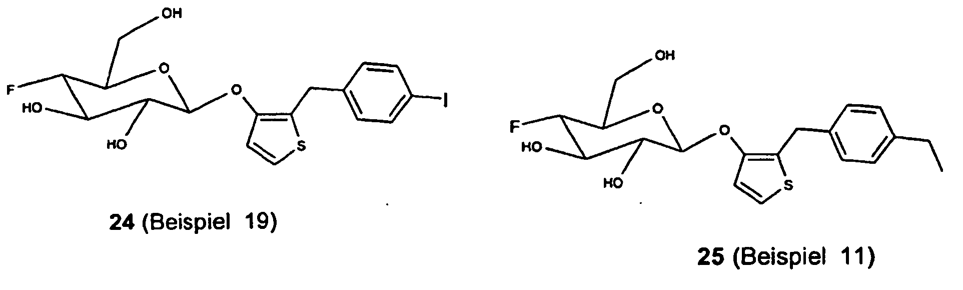

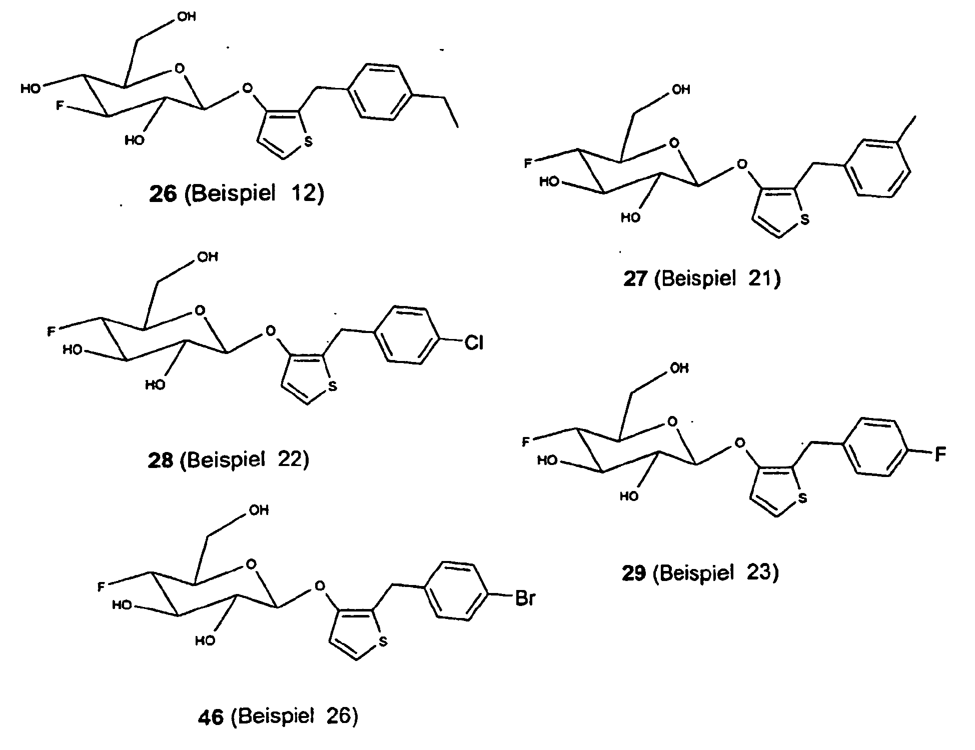

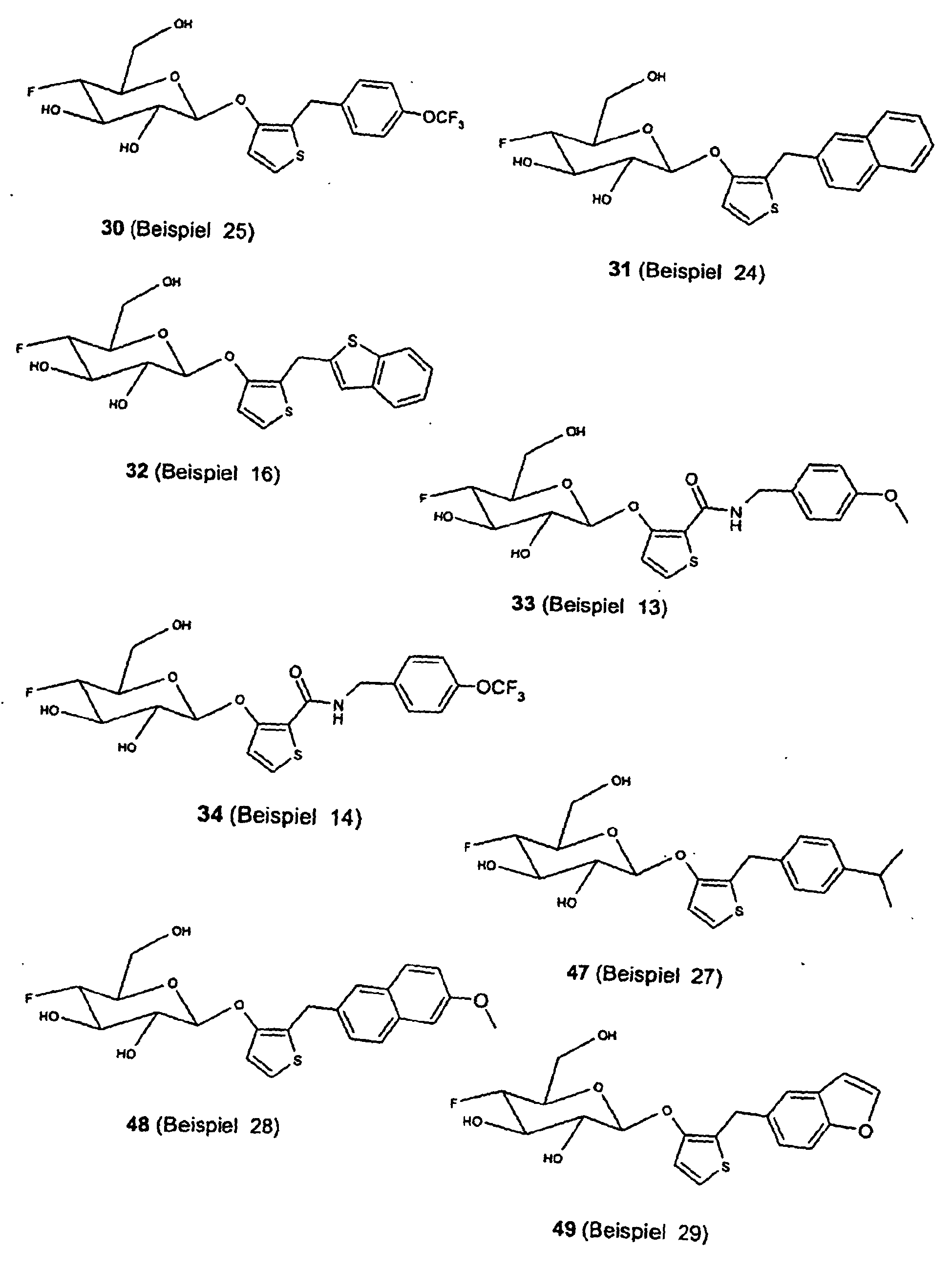

### Beispiel 1 (Verbindung 17)

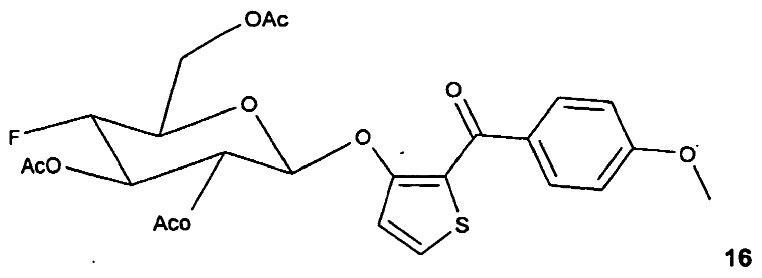

400 mg (1.7 mmol) (3-Hydroxy-thiophen-2-yl)-(4-methoxy-phenyl)-methanon (**15**) (DE-Anmeldenummer 10231370.9 (2002/0049)) und 200 mg (0.54 mmol) Bromid **2** werden in 6 ml Metylenchlorid gelöst. Zu dieser Lösung werden nacheinander 160 mg Bu₃BnNCl (PTK = Phasentransferkatalysator), 320 mg K₂CO₃ und 0.4 ml Wasser zugegeben und anschließend 20 Stunden bei Raumtemperatur gerührt. Die Reaktionslösung wird mit 20 ml Ethylacetat verdünnt und über Kieselgel filtriert. Das Filtrat wird eingeengt und der Rückstand wird durch Chromatographie über Kieselgel (Ethylacetat/Heptan = 1/1) getrennt. Man erhält 160 mg (56 %) **16** als farblosen Feststoff. C₂₄H₂₅FO₁₀S (524.52) MS (ESI⁺) 525.12 (M+H⁺).

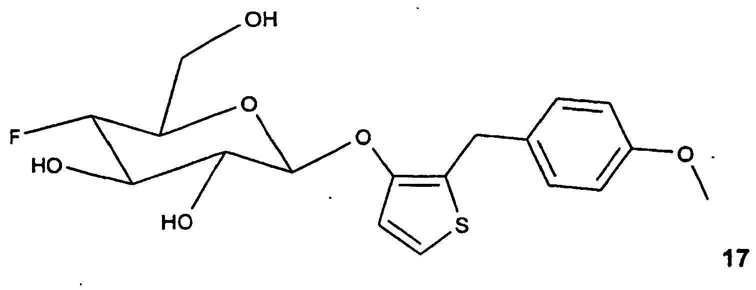

150 mg (0.29 mmol) der Verbindung **16** werden in 4 ml Acetonitril gelöst. Diese Lösung wird im Eisbad gekühlt und dann werden 150 mg NaCNBH₃ und 0.2 ml TMSCI zugegeben. Anschließend wird die Kühlung entfernt und noch 2 Stunden bei Raumtemperatur gerührt. Die Reaktionslösung wird mit 20 ml Ethylacetat verdünnt und über Kieselgel filtriert. Das Filtrat wird eingeengt und man erhält 150 mg Rohprodukt. Dieses Rohprodukt wird in 4 ml Methanol aufgenommen und mit 1 ml 1 M NaOMe in MeOH versetzt. Nach einer Stunde wird mit methanolischer HCl neutralisiert, eingeengt und der Rückstand wird durch Chromatographie an Kieselgel (Methylenchlorid/Methanol/konz.Amoniak, 30/5/1) gereinigt. Man erhält 76 mg (69 % über 2 Stufen) 17 als farblosen Feststoff. C₁₈H₂₁FO₆S (384.43) MS (ESI⁺) 403.21 (M + H₂O + H⁺).

### Beispiel 2 (Verbindung 18)

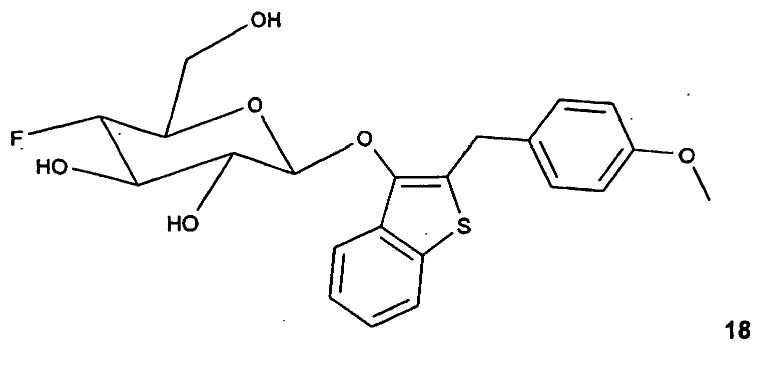

100 mg (0.47 mmol) (3-Hydroxy-benzothiophen-2-yl)-(4-methoxy-phenyl)-methanon (Eur. J. Med. Chem. 1985, 20, 187-189) und 300 mg (0.80 mmol) Bromid 2 werden in 10 ml Chloroform gelöst. Zu dieser Lösung werden nacheinander 120 mg Bu₃BnNCl (PTK = Phasentransferkatalysator) und 1.5 ml 1 N wässrige Natronlauge zugegeben und anschließend 4 Stunden am Rückfluss gekocht. Die Reaktionslösung wird mit 20 ml Ethylacetat verdünnt und über Kieselgel filtriert. Das Filtrat wird eingeengt und der Rückstand wird durch Chromatographie an Kieselgel (Ethylacetat/Heptan = 1/1) getrennt. Man erhält 135 mg (51 %) hellgelben Feststoff. Dieser wird analog der Herstellung von Verbindung **17** mit 100 mg NaCNBH₃ und 0.2 ml TMSCI und dann mit NaOMe/MeOH zu Verbindung **18** umgesetzt. Man erhält 46 mg **18.** C₂₂H₂₃FO₆S (434.49) MS (ESI⁻) 479.18 (M + CHO₂⁻).

### Beispiel 3 (Verbindung 19)

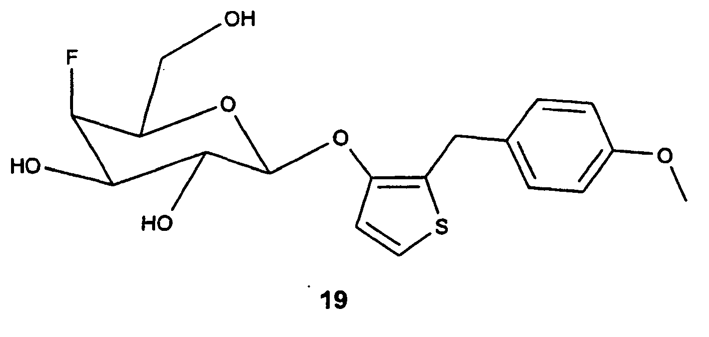

178 mg (3-Hydroxy-thiophen-2-yl)-(4-methoxy-phenyl)-methanon (**15**) und 90 mg Bromid **4** werden analog der Synthese von Beispiel 1 umgesetzt und man erhält 49 mg **19** als farblosen Feststoff. C₁₈H₂₁FO₆S (384.43) MS (ESI⁺) 403.21 (M+H₂O+H⁺).

### Beispiel 4 (Verbindung 20)

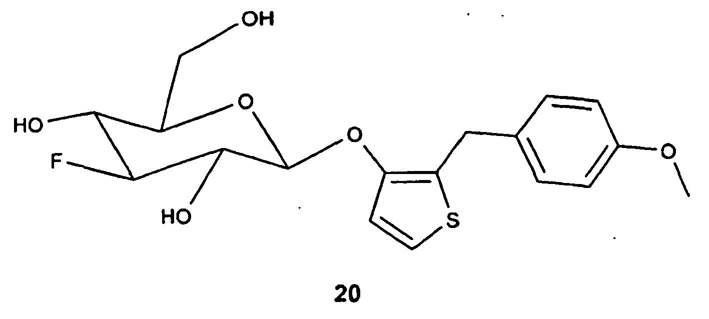

200 mg (3-Hydroxy-thiophen-2-yl)-(4-methoxy-phenyl)-methanon **15** und 100 mg Bromid 6 werden analog der Synthese von Beispiel 1 umgesetzt und man erhält 59 mg 20 als farblosen Feststoff. C₁₈H₂₁FO₆S (384.43) MS (ESI⁺) 403.21 (M+H₂O+H⁺).

Die Synthese der Beispiele **11** (Verbindung **25**) und **15** (Verbindung **21**) erfolgt analog der Synthese von Beispiel **1** ausgehend von den entsprechenden Hydroxythiophenen und dem Bromid **2.**

Die Synthese der Beispiele **16** (Verbindung **32**)**,** 17 (Verbindung **23**), 18 (Verbindung **22**), **19** (Verbindung **24**), 21 (Verbindung **27**), 22 (Verbindung **28**), 23 (Verbindung **29**), 24 (Verbindung **31**), 25 (Verbindung 30), 26 (Verbindung **46**), 27 (Verbindung **47**), 28 (Verbindung **48**), und 29 (Verbindung **49**) erfolgt analog der Synthese von Beispiel **1** ausgehend von entsprechenden Hydroxythiophenen und dem Bromid **14.**

Die Synthese des Beispiels 12 (Verbindung **26**) erfolgt analog der Synthese von Beispiel 4 ausgehend vom entsprechenden Hydroxythiophen und Bromid **6.**

Die Synthese der Beispiele 13 (Verbindung **33**) und 14 (Verbindung **34**) erfolgt analog der Synthese von Verbindung **16** durch Umsetzung der entsprechenden Hydroxythiophene mit dem Bromid 2 und anschließendem Entschützen mit NaOMe/MeOH analog zu Beispiel 1.

Die Synthese des Beispiels 20 (Verbindung **35**) erfolgt analog der Synthese von Beispiel 1 ausgehend von Hydroxythiophen **15** und dem Bromid **10.**

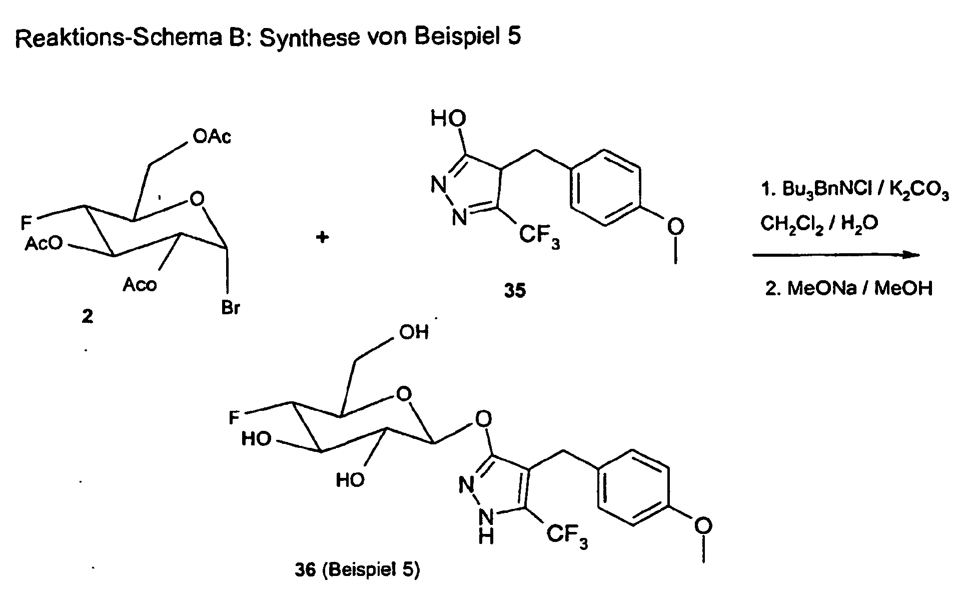

### Weitere Beispiel-Verbindungen:

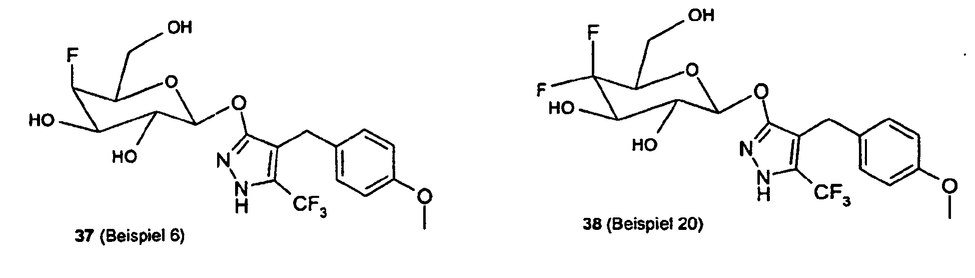

### Beispiel 5 (Verbindung 36)

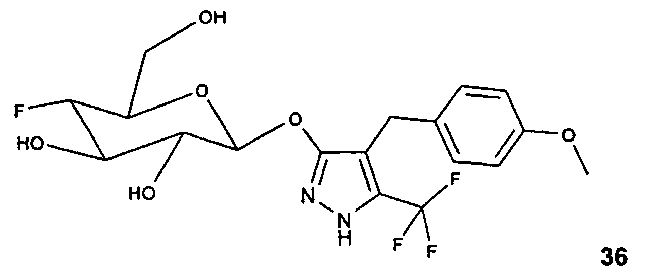

200 mg 4-(4-Methoxy-benzyl)-5-methyl-1 H-pyrazol-3-ol (**35**) (J. Med. Chem. 1996, 39, 3920-3928) werden analog der Synthese von Beispiel 1 mit 100 mg des Bromids **2** glykosiliert und dann analog Beispiel 1 mit NaOMe/MeOH entschützt. Man erhält 49 mg der Verbindung **36** als farblosen Feststoff. C₁₈H₂₀F₄N₂O₆ (436.36) MS (ESI⁺) 437.21 (M + H⁺).

### Beispiel 6 (Verbindung 37)

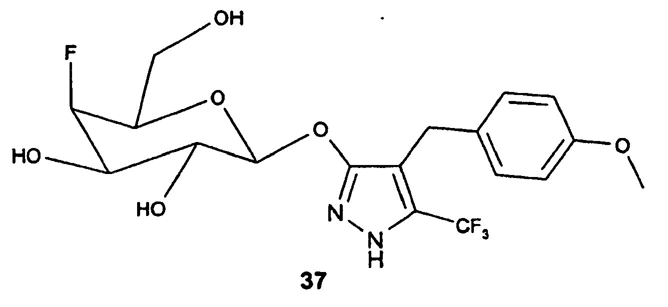

200 mg 4-(4-Methoxy-benzyl)-5-methyl-1H-pyrazol-3-ol (35) und 100 mg Bromid **4** werden analog der Synthese von Beispiel 1 glykosiliert und dann analog Beispiel 1 mit NaOMe/MeOH entschützt. Man erhält 89 mg der Verbindung **37** als farblosen Feststoff. C₁₈H₂₀F₄N₂O₆ (436.36) MS (ESI⁺) 437.21 (M + H⁺).

### Beispiel 20 (Verbindung 38)

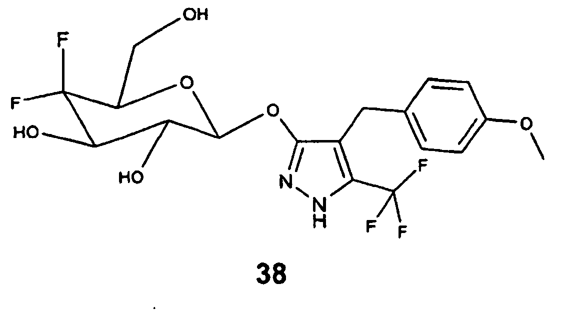

110 mg 4-(4-Methoxy-benzyl)-5-methyl-1H-pyrazol-3-ol (35) und 60 mg Bromid **10** werden analog der Synthese von Beispiel **1** glykosiliert und dann analog Beispiel **1** mit NaOMe/MeOH entschützt. Man erhält 49 mg der Verbindung **38** als farblosen Feststoff. C₁₈H₁₉F₅N₂O₆ (454.35) MS (ESI⁺) 455.22 (M + H⁺).

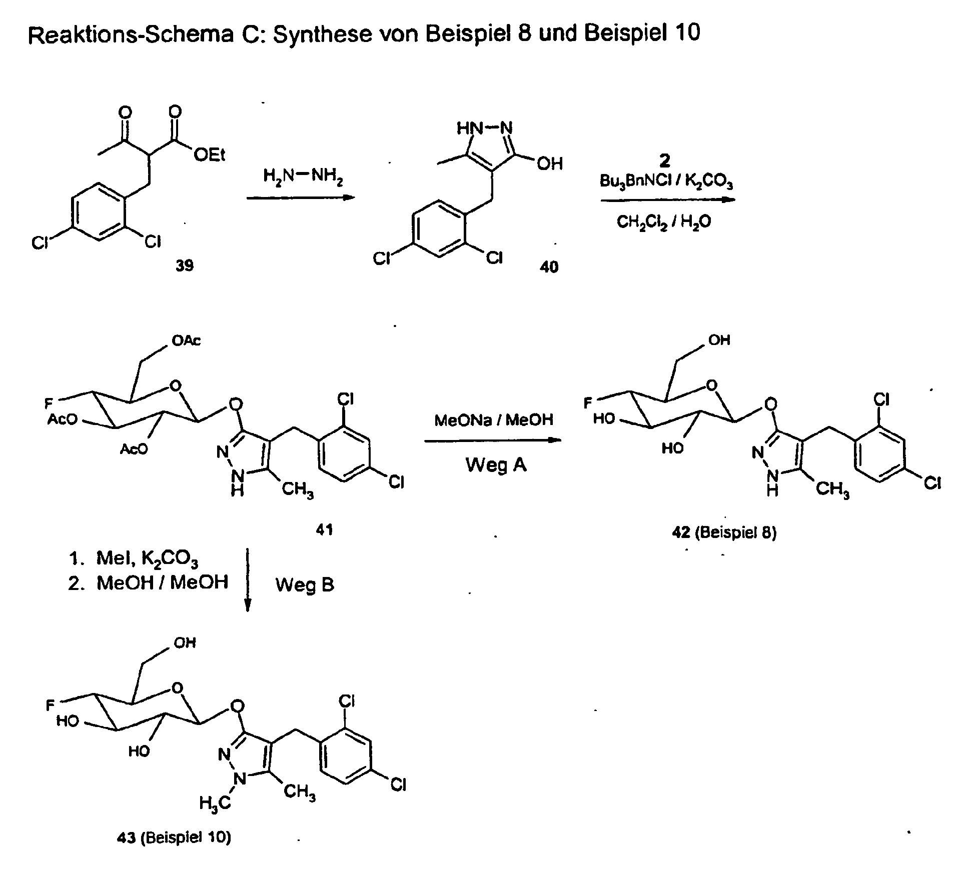

### Weitere Beispiel-Verbindungen:

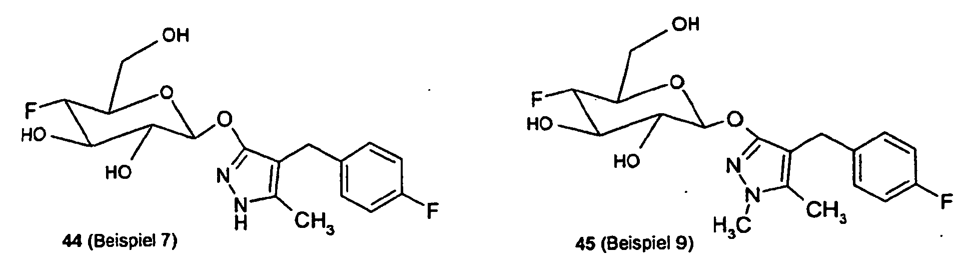

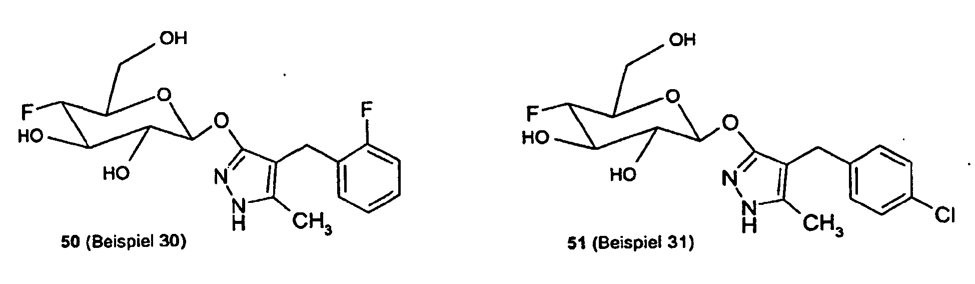

### Beispiel 8 (Verbindung 42)

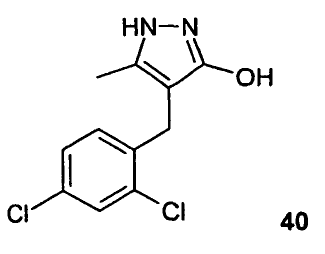

500 mg (1,73 mmol) 2-(2,4-Dichlorbenzyl)-3-oxobuttersäureethylester (**39**) (Bionet) werden mit 0,21 ml Hydrazinhydrat 51%ig (3,46 mmol) in 15 ml Toluol für 1,5 h am Wasserabscheider gekocht. Nach dem Erkalten wird der Feststoff abgesaugt und mit Toluol und Ether nachgewaschen. Man erhält 400 mg (90%) der Verbindung 40 als weißen voluminösen Niederschlag. C₁₁H₁₀C₁₂N₂O (257,12) MS (ESI): 257 (M+H⁺).

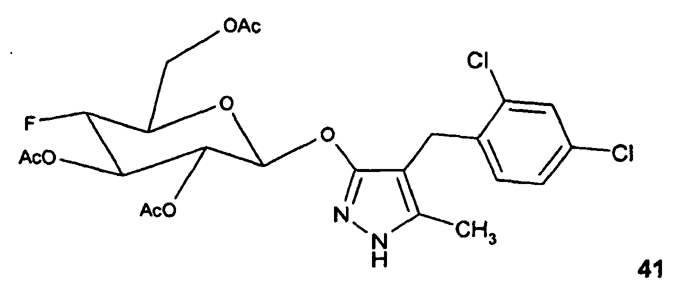

270 mg (1,05 mmol) 4-(2,4-Dichlor-benzyl)-5-methyl-1H-pyrazol-3-ol (**40**) wurden in 25 ml Methylenchlorid gelöst und mit 0,7 ml Wasser, 1,2 g (8,68 mmol) Kaliumcarbonat, 84 mg (0,31 mmol) Benzyltriethylammoniumbromid und 428 mg (1,15 mmol) Bromid **2** versetzt und 18 h bei RT gerührt. Die Reaktionslösung wurde mit Methylenchlorid verdünnt und je einmal mit Wasser und gesättigter Kochsalzlösung gewaschen, über MgSO₄ getrocknet und eingeengt. Das Rohprodukt wurde über Kieselgel gereinigt. Man erhält 122 mg (21%) der Verbindung **41** als eines weißen Feststoff.
C₂₃H₂₅Cl₂FN₂O₈ (547,37) MS (ESI): 547 (M+H⁺).

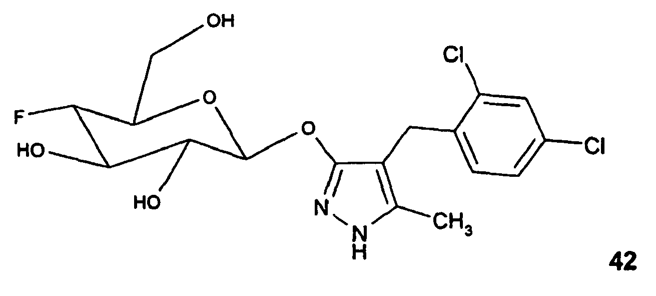

70 mg (0,1278 mmol) der Verbindung **41** werden entsprechend des Wegs A in 2 ml Methanol gelöst und mit 1,02 ml (0,511 mmol) Natriummethanolatlösung (0,5 M) in Tetrahydrofuran versetzt. Nach 5 Min. werden 27,6 mg (0,516 mmol) Ammonchlorid und 2,0 g SiO₂ zugegeben. Die Lösung wird eingeengt und das Produkt wird über Kieselgel filtriert und erst mit EtOAc und anschließend mit EtOAc/Methanol 20:1 nachgewaschen. Man erhält 50 mg (90%) der Verbindung **42** als farblosen Feststoff. C₁₇H₁₉C₁₂FN₂O₅ (421,26) MS (ESI): 420 (M+H⁺).

### Beispiel 10 (Verbindung 43)

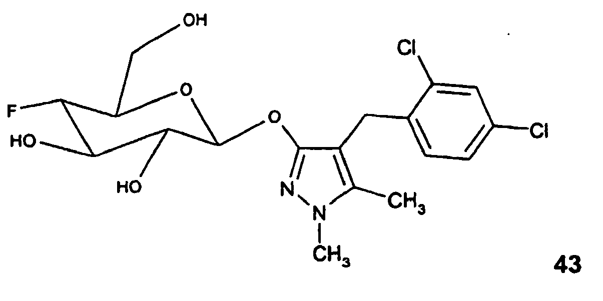

50 mg der Verbindung **41** werden entsprechend des Wegs B in 2,0 ml DMF gelöst und bei Raumtemperatur mit 50 mg K₂CO₃ und 57 µl Methyliodid versetzt. Nach 14 Tagen werden 30 ml EtOAc zugegeben und die organische Phase wird zweimal mit je 20 ml H₂O gewaschen und eingeengt. Das Rohprodukt wird säulenchromatographisch (EtOAc/Heptan = 3:1) gereinigt und analog zur Darstellung von Verbindung **42** mit NaOMe / MeOH umgesetzt. Man erhält 9,1 mg der Verbindung **43** als farbloses Wachs. C₁₈H₂₁C₁₂FN₂O₅ (435,24) MS (ESI): 434 (M+H⁺).

Die Synthese der Beispiele 7 (Verbindung **44**), 30 (Verbindung **50**) und 31 (Verbindung **51**) erfolgt analog der beschriebenen Synthese des Beispiels 8 (Verbindung **42**) ausgehend von den entsprechenden β-Ketoestem.

Die Synthese des Beispiels 9 (Verbindung **45**) erfolgt analog der beschriebenen Synthese des Beispiels 10 (Verbindung **43**) ausgehend vom entsprechenden β-Ketoester.

## Patentansprüche

1. Verbindungen der Formel I, 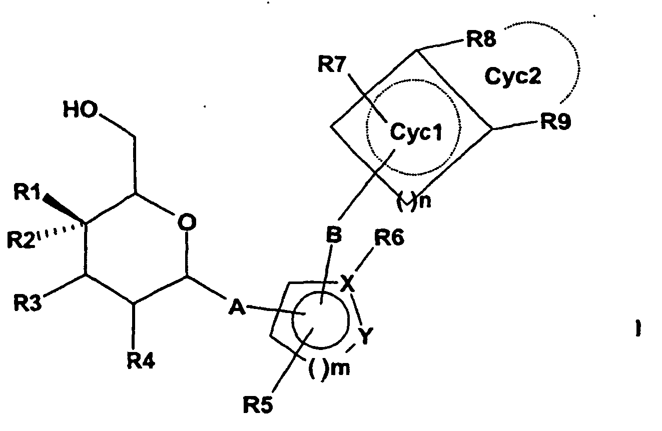 worin bedeuten
R1 und R2 unabhängig voneinander F, H oder einer der Reste R1 oder R2 OH;
R3 OH oder F, wobei mindestens einer der Reste R1, R2, R3 F sein muss;
R4 OH;
A O, NH, CH₂, S oder eine Bindung;
X C, O, S oder N, wobei im Falle Y = O oder S, X = C sein muss;
Y N, O oder S;
m eine Zahl 1 oder 2;
R5 Wasserstoff, F, Cl, Br, J, OH, CF₃, NO₂, CN, COOH, CO(C₁-C₆)-Alkyl, COO(C₁-C₆)-Alkyl, CONH₂, CONH(C₁-C₆)-Alkyl, CON[(C₁-C₆)-Alkyl]₂, (C₁₋C₆)-Alkyl, (C₂-C₆)-Alkenyl, (C₂-C₆)-Alkinyl, (C₁-C₆)-Alkoxy, HO-(C₁-C₆)-Alkyl, (C₁-C₆)-Alkyl-O-(C₁-C₆)-alkyl, Phenyl, Benzyl, (C₁-C₆)-Alkoxycarboxyl, wobei in den Alkyl-, Alkoxy-, Alkenyl- oder Alkinylresten ein, mehrere, oder alle Wasserstoff(e) durch Fluor ersetzt sein können; SO₂-NH₂, SO₂NH(C₁-C₆)-Alkyl, SO₂N[(C₁-C₆)-Alkyl]₂, S-(C₁-C₆)-Alkyl, S-(CH₂)ₒ-Phenyl, SO-(C₁-C₆)-Alkyl, SO-(CH₂)ₒ-Phenyl, SO₂-(C₁-C₆)-Alkyl, SO₂-(CH₂)ₒ-Phenyl, wobei o = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)2, NH(C₁-C₇)-Acyl, Phenyl, O-(CH₂)ₒ-Phenyl, wobei o = 0 - 6 sein kann, wobei der Phenylring ein bis 3-fach substituiert sein kann mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂₋CH₃, COOH, COO-(C₁-C₆)-Alkyl, CONH₂;
oder im Falle Y = S R5 und R6 gemeinsam mit den sie tragenden C-Atomen Phenyl;
R6 gegebenenfalls H, (C₁-C₆)-Alkyl, (C₁-C₆)-Alkenyl, (C₃-C₆)-Cycloalkyl, oder Phenyl, dass gegebenenfalls durch Halogen oder (C₁-C₄)-Alkyl substituiert sein kann;
B (C₀-C₁₅)-Alkandiyl, wobei ein oder mehrere C-Atome des Alkandiyl-Rests unabhängig voneinander durch -O-, -(C=O)-, -CH=CH-, -C≡C-, -S-, -CH(OH)-, -CHF-, -CF₂-, -(S=O)-, -(SO₂)-, -N((C₁-C₆)-Alkyl)-, -N((C₁-C₆)-Alkyl-Phenyl)- oder -NH- ersetzt sein können;
n eine Zahl von 0 bis 4;
Cyc1 ein 3 bis 7 gliedriger gesättigter, teilweise gesättigter oder ungesättigter Ring, wobei 1 C-Atom durch O, N oder S ersetzt sein kann;
R7, R8, R9 Wasserstoff, F, Cl, Br, J, OH, CF₃, NO₂, CN, COOH, COO(C₁-C₆)-Alkyl, CO(C₁-C₄)-Alkyl, CONH₂, CONH(C₁-C₆)-Alkyl, CON[(C₁-C₆)-Alkyl]₂, (C₁₋C₆)-Alkyl, (C₂-C₆)-Alkenyl, (C₂-C₆)-Alkinyl, (C₁-C₈)-Alkoxy, HO-(C₁-C₆)-Alkyl, (C₁-C₆)-Alkyl-O-(C₁-C₆)-alkyl, wobei in den Alkyl-, Alkoxy-, Alkenyl- oder Alkinylresten ein, mehrere, oder alle Wasserstoff(e) durch Fluor ersetzt sein können;
SO₂-NH₂, SO₂NH(C₁-C₆)-Alkyl, SO₂N[(C₁-C₆)-Alkyl]₂ , S-(C₁-C₆)-Alkyl, S-(CH₂)ₒ-Phenyl, SCF₃, SO-(C₁-C₆-Alkyl, SO-(CH₂)ₒ-Phenyl, SO₂-(C₁-C₆)-Alkyl, SO₂-(CH₂)ₒ-Phenyl, wobei o = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH(C₁-C₇)-Acyl, Phenyl, O-(CH₂)ₒ-Phenyl, wobei o = 0 - 6 sein kann, wobei der Phenylring ein bis 3-fach substituiert sein kann mit F, Cl, Br, J, OH, CF₃, NO₂, CN, OCF₃, (C₁₋C₈)-Alkoxy, (C₁-C₆)-Alkyl, NH₂, NH(C₁-C₆)-Alkyl, N((C₁-C₆)-Alkyl)₂, SO₂₋CH₃, COOH, COO-(C₁-C₆)-Alkyl, CONH₂;
oder
R8 und R9 gemeinsam mit den sie tragenden C-Atomen ein 5 bis 7 gliedrigen, gesättigter, teilweise oder vollständig ungesättigter Ring Cyc2, wobei 1 oder 2 C-Atom(e) des Ringes auch durch N, O oder S ersetzt sein können und Cyc2 gegebenenfalls durch (C₁-C₆)-Alkyl, (C₂-C₅)-Alkenyl, (C₂-C₅)-Alkinyl, wobei jeweils eine CH₂-Gruppe durch O ersetzt sein kann, oder durch H, F, Cl, OH, CF₃, NO₂, CN, COO(C₁-C₄)-Alkyl, CONH₂, CONH(C₁₋C₄)-Alkyl, OCF₃ substituiert sein kann;
sowie deren pharmazeutisch verträglichen Salze.

2. Verbindungen der Formel gemäß Anspruch 1, worin bedeuten
R1 und R2 unabhängig voneinander F oder H und einer der Reste R1 oder R2 = OH, wobei einer der Reste R1oder R2 F sein muss;
R3 OH;
R4 OH;
A O oder NH;
X C, O oder N, wobei im Falle Y = S, X = C sein muss;
Y S oder N;
m eine Zahl 1 oder 2;
R5 Wasserstoff, F, Cl, Br, J, OH, CF₃, NO₂, CN, COOH, CO(C₁-C₆)-Alkyl, COO(C₁-C₆)-Alkyl, CONH₂, CONH(C₁-C₆)Alkyl, CON[(C₁-C₆)-Alkyl]₂, (C₁₋C₆)-Alkyl, (C₂-C₆)-Alkenyl, (C₂-C₆)-Alkinyl, (C₁-C₆)-Alkoxy, HO-(C₁- C₆)-Alkyl, (C₁-C₆)-Alkyl-O-(C₁-C₆)-alkyl, Phenyl, Benzyl, (C₁-C₄)-Alkylcarboxyl, SO-(C₁-C₆)-Alkyl, wobei in den Alkyl- oder Alkoxyresten ein, mehrere, oder alle Wasserstoff(e) durch Fluor ersetzt sein können; oder im Falle Y = S R5 und R6 gemeinsam mit den sie tragenden C-Atomen Phenyl;
R6 gegebenenfalls H, (C₁-C₆)-Alkyl, (C₁-C₆)-Alkenyl, (C₃-C₆)-Cycloalkyl, oder Phenyl, das gegebenenfalls durch Halogen oder (C₁-C₄)-Alkyl substituiert sein kann;
B (C₀-C₁₅)-Alkandiyl, wobei ein oder mehrere C-Atom(e) des Alkandiyl-Rests unabhängig voneinander durch -O-, -(C=O)-, -CH=CH-, -C≡C-, -S-, -CH(OH)-, -CHF-, -CF₂-, -(S=O)-, -(SO₂)-, -N((C₁-C₆)-Alkyl)-, -N((C₁-C₆)-Alkyl-Phenyl)- oder-NH- ersetzt sein können;
n eine Zahl von 0 bis 4;
Cyc1 ein 3 bis 7 gliedriger gesättigter, teilweise gesättigter oder ungesättigter Ring, wobei 1 C-Atom durch O oder S ersetzt sein kann;
R7, R8, R9 Wasserstoff, F, Cl, Br, J, OH, CF₃, NO₂, CN, COOH, COO(C₁-C₆)-Alkyl, CO(C₁-C₄)-Alkyl, CONH₂, CONH(C₁-C₆)-Alkyl, CON[(C₁-C₆)-Alkyl]₂, (C₁₋C₆)-Alkyl, (C₂-C₆)-Alkenyl, (C₂-C₆)-Alkinyl, (C₁-C₈)-Alkoxy, HO-(C₁-C₆)-Alkyl, (C₁-C₆)-Alkyl-O-(C₁-C₆)-alkyl, S-(C₁-C₆)-Alkyl, SCF₃, SO-(C₁-C₆)-Alkyl, wobei in den Alkyl- bzw. Alkoxyresten ein, mehrere, oder alle Wasserstoff(e) durch Fluor ersetzt sein können;
oder
R8 und R9 gemeinsam mit den sie tragenden C-Atomen ein 5 bis 7 gliedrigen, gesättigter, teilweise oder vollständig ungesättigter Ring Cyc2, wobei 1 oder 2 C-Atom(e) des Ringes auch durch N, O oder S ersetzt sein können und Cyc2 gegebenenfalls durch (C₁-C₆)-Alkyl, (C₂-C₅)-Alkenyl, (C₂-C₅)-Alkinyl, wobei jeweils eine CH₂-Gruppe durch O ersetzt sein kann, oder durch H, F, Cl, OH, CF₃, NO₂, CN, COO(C₁-C₄)-Alkyl, CONH₂, CONH(C₁₋C₄)-Alkyl, OCF₃ substituiert sein kann.

3. Verbindungen der Formel I gemäß den Ansprüchen 1 oder 2, worin die Zucker-Reste beta(β)-verknüpft sind und die Stereochemie in 2-, 3- und 5-Position des Zuckerrestes D-gluco-konfiguriert ist.

4. Verbindungen der Formel I gemäß den Ansprüchen 1 bis 3, worin
R1 und R2 unabhängig voneinander F, H oder einer der Reste R1 oder R2 = OH, wobei mindestenseiner der Reste R1 oder R2 F sein muss;
R3 OH;
R4 OH;
A O;
X C, O oder N, wobei im Falle Y = S, X = C sein muss;
Y S oder N;
m eine Zahl 1;
R5 Wasserstoff,; (C₁-C₅)-Alkyl, (C₁-C₄)-Alkoxy, HO-(C₁-C₄)-Alkyl, (C₁-C₄)-Alkyl-O-(C₁-C₄)-alkyl, F, Cl, CF₃, OCF₃, OCH₂CF₃ (C₁-C₄)-Alkyl-CF₂-, Phenyl, Benzyl, (C₁-C₄)-Alkylcarboxyl, (C₂-C₄)-Alkenyl, (C₂-C₄)-Alkinyl, COO(C₁-C₄)-Alkyl;
oder
im Falle Y = S R5 und R6 gemeinsam mit den sie tragenden C-Atomen Phenyl;
R6 gegebenenfalls H, (C₁-C₆)-Alkyl, (C₁-C₆)-Alkenyl, (C₃-C₆)-Cycloalkyl, oder Phenyl, das gegebenenfalls durch Halogen oder (C₁-C₄)-Alkyl substituiert sein kann;
B (C₁-C₄)-Alkandiyl, wobei eine CH₂-Gruppe auch ersetzt sein kann durch -(C=O)-, -CH(OH)-, -CO-NH-, -CHF-, -CF₂-, -O-;
n eine Zahl 2 oder 3;
Cyc1 ein ungesättigter 5 bis 6 gliedriger Ring, wobei 1 C-Atom durch O oder S ersetzt sein kann;
R7, R8, R9 Wasserstoff, (C₁-C₄)-Alkyl, (C₁-C₈)-Alkoxy, S-(C₁-C₄)-Alkyl, SCF₃, F, Cl, Br, J, OCF₃, OCH₂CF₃ , OH , HO-(C₁-C₄)-Alkyl, (C₁-C₄)-Alkyl-O-(C₁-C₄)-alkyl, oder
R8 und R9 gemeinsam -CH=CH-O-, -CH=CH-S-, -CH=CH-CH=CH-, das gegebenenfalls durch (C₁-C₄)-Alkoxy substituiert ist, oder -O-(CH₂)ₚ-O-, mit p = 1 oder 2 und
R7 Wasserstoff bedeuten.

5. Verbindungen der Formel I gemäß den Ansprüchen 1 bis 4, worin
R1, R2 H oder F, wobei einer der Reste R1, R2 F sein muss;
R3 OH;
R4 OH;
A O;
X C und Y = S, oder
X O und Y = N, oder
X N und Y = N;
m eine Zahl 1;
R5 Wasserstoff, CF₃, (C₁-C₆)-Alkyl, oder im Falle Y = S R5 und R6 gemeinsam mit den sie tragenden C-Atomen Phenyl;
R6 gegebenenfalls H, (C₁-C₄)-Alkyl oder Phenyl;
B -CH₂-, -C₂H₄-, -C₃H₆, -CO-NH-CH₂- oder -CO-CH₂-CH₂-;
n eine Zahl 2 oder 3;
Cyc1 ungesättigter 5 bis 6-gliedriger Ring, wobei 1 C-Atom durch S ersetzt sein kann;
R7,R8,R9 Wasserstoff, (C₁-C₆)-Alkyl, (C₁-C₄)-Alkoxy, S-(C₁-C₄)-Alkyl, SCF₃, F, Cl, Br, J, OCF₃ oder
R8 und R9 gemeinsam -CH=CH-O-, -CH=CH-CH=CH-, das gegebenenfalls durch (C₁-C₄)-Alkoxy substituiert ist, und
R7 Wasserstoff bedeuten.

6. Verbindungen der Formel I gemäß den Ansprüchen bis 5, worin
R1 ,R2 H oder F, wobei einer der Reste R1 oder R2 F bedeutet;
R3 OH;
R4 OH;
A O;
X C und Y = S oder
X N und Y = N;
m eine Zahl 1;
R5 Wasserstoff, (C₁-C₄)-Alkyl oder CF₃ oder im Falle Y = S R5 und R6 gemeinsam mit den sie tragenden C-Atomen Phenyl;
R6 gegebenenfalls H oder (C₁-C₄)-Alkyl ;
B -CH₂- oder -CO-NH-CH₂-;
n eine Zahl 2 oder 3;
Cyc1 Phenyl oder Thiophen;
R7 Wasserstoff, Methoxy, F, Cl, Br, J, (C₁-C₄)-Alkyl, OCF₃;
R8,R9 Wasserstoff oder Cl oder
R8 und R9 zusammen mit den sie tragenden C-Atomen Phenyl, das gegebenenfalls durch Methoxy substituiert sein kann, oder Furan und
R7 Wasserstoff bedeuten.

7. Arzneimittel enthaltend eine oder mehrere der Verbindungen gemäß einem oder mehreren der Ansprüche 1 bis 6.

8. Arzneimittel enthaltend eine oder mehrere der Verbindungen gemäß einem oder mehreren der Ansprüche 1 bis 6 und ein oder mehrere Blutzucker senkende Wirkstoffe.

9. Verwendung der Verbindungen gemäß einem oder mehreren der Ansprüche 1 bis 6 zur Herstellung eines Medikamentes zur Behandlung des Typ 1 und Typ 2 Diabetes.

10. Verwendung der Verbindungen gemäß einem oder mehreren der Ansprüche 1 bis 6 zur Herstellung eines Medikamentes zur Blutzuckersenkung.

11. Verwendung der Verbindungen gemäß einem oder mehreren der Ansprüche 1 bis 6 in Kombination mit mindestens einem weiteren Blutzucker senkenden Wirkstoff zur Herstellung eines Medikamentes zur Behandlung des Typ 1 und Typ 2 Diabetes.

12. Verwendung der Verbindungen gemäß einem oder mehreren der Ansprüche 1 bis 6 in Kombination mit mindestens einem weiteren Blutzucker senkenden Wirkstoff zur Herstellung eines Medikamentes zur Blutzuckersenkung.

13. Verfahren zur Herstellung eines Arzneimittels enthaltend eine oder mehrere der Verbindungen gemäß einem oder mehreren der Ansprüche 1 bis 6, **dadurch gekennzeichnet, dass** der Wirkstoff mit einem pharmazeutisch geeigneten Träger vermischt wird und diese Mischung in eine für die Verabreichung geeignete Form gebracht wird.

## Claims

1. A compound of the formula I 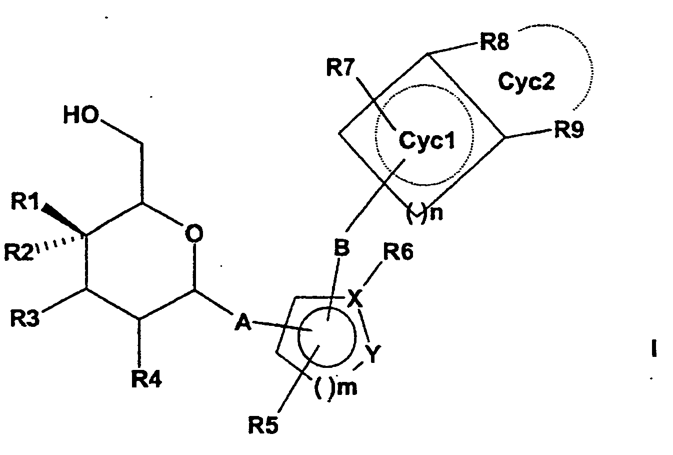 in which the meanings are
R1 and R2 independently of one another F, H or one of the radicals R1 or R2 OH;
R3 OH or F, where at least one of the radicals R1, R2, R3 must be F;
R4 OH;
A O, NH, CH₂, S or a bond;
X C, O, S or N, where X must be C when Y is O or S;
Y N, O or S;
m a number 1 or 2;
R5 hydrogen, F, Cl, Br, I, OH, CF₃, NO₂, CN, COOH, CO(C₁-C₆)-alkyl, COO(C₁-C₆)-alkyl, CONH₂, CONH(C₁-C₆)-alkyl, CON[(C₁-C₆)-alkyl]₂, (C₁-C₆)-alkyl, (C₂-C₆)-alkenyl, (C₂-C₆)-alkynyl, (C₁-C₆)-alkoxy, HO-(C₁-C₆)-alkyl, (C₁-C₆)-alkyl-O-(C₁-C₆)-alkyl, phenyl, benzyl, (C₁-C₆)-alkoxycarboxyl, it being possible for one, more than one or all hydrogen(s) in the alkyl, alkoxy, alkenyl or alkynyl radicals to be replaced by fluorine;
SO₂-NH₂, SO₂NH(C₁-C₆)-alkyl, SO₂N[(C₁-C₆)-alkyl]₂, S-(C₁-C₆)-alkyl, S-(CH₂)ₒ-phenyl, SO-(C₁-C₆)-alkyl, SO-(CH₂)ₒ-phenyl, SO₂-(C₁-C₆)-alkyl, SO₂-(CH₂)ₒ-phenyl, where o can be 0-6, and the phenyl radical may be substituted up to twice by F, Cl, Br, OH, CF₃, NO₂, CN, OCF₃, O-(C₁-C₆)-alkyl, (C₁-C₆)-alkyl, NH₂;
NH₂, NH-(C₁-C₆)-alkyl, N((C₁-C₆)-alkyl)₂, NH(C₁-C₇)-acyl, phenyl, O-(CH₂)ₒ-phenyl, where o can be 0-6, where the phenyl ring may be substituted one to 3 times by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
or when Y is S, R5 and R6 together with the C atoms carrying them phenyl;
R6 optionally H, (C₁-C₆)-alkyl, (C₁-C₆)-alkenyl, (C₃-C₆)-cycloalkyl, or phenyl that may optionally be substituted by halogen or (C₁-C₄)-alkyl;
B (C₀-C₁₅)-alkanediyl, it being possible for one or more C atoms in the alkanediyl radical to be replaced independently of one another by -O-, -(C=O)-, -CH=CH-, -C≡C-, -S-, -CH(OH)-, -CHF-, -CF₂-, -(S=O)-, -(SO₂)-, -N((C₁-C₆)-alkyl)-, -N((C₁-C₆)-alkyl-phenyl)- or -NH-;
n a number from 0 to 4;
Cyc1 a 3 to 7 membered saturated, partially saturated or unsaturated ring, where 1 C atom may be replaced by O, N or S;
R7, R8, R9 hydrogen, F, Cl, Br, I, OH, CF₃, NO₂, CN, COOH, COO(C₁-C₆)-alkyl, CO(C₁-C₄)-alkyl, CONH₂, CONH(C₁-C₆)-alkyl, CON[(C₁-C₆)-alkyl]₂, (C₁-C₆)-alkyl, (C₂-C₆)-alkenyl, (C₂-C₆)-alkynyl, (C₁-C₈)-alkoxy, HO-(C₁-C₆)-alkyl, (C₁-C₆)-alkyl-O-(C₁-C₆)-alkyl, it being possible for one, more than one or all hydrogen(s) in the alkyl, alkoxy, alkenyl or alkynyl radicals to be replaced by fluorine;
SO₂-NH₂, SO₂NH(C₁-C₆)-alkyl, SO₂N[(C₁-C₆)-alkyl]₂, S-(C₁-C₆)-alkyl, S-(CH₂)ₒ-phenyl, SCF₃, SO-(C₁-C₆)-alkyl, SO-(CH₂)ₒ-phenyl, SO₂-(C₁-C₆)-alkyl, SO₂-(CH₂)ₒ-phenyl, where o can be 0-6, and the phenyl radical may be substituted up to twice by F, Cl, Br, OH, CF₃, NO₂, CN, OCF₃, O-(C₁-C₆)-alkyl, (C₁-C₆)-alkyl, NH₂;
NH₂, NH-(C₁-C₆)-alkyl, N((C₁-C₆)-alkyl)₂, NH(C₁-C₇)-acyl, phenyl, O-(CH₂)ₒ-phenyl, where o can be 0-6, where the phenyl ring may be substituted one to 3 times by F, Cl, Br, I, OH, CF₃, NO₂, CN, OCF₃, (C₁-C₈)-alkoxy, (C₁-C₆)-alkyl, NH₂, NH(C₁-C₆)-alkyl, N((C₁-C₆)-alkyl)₂, SO₂-CH₃, COOH, COO(C₁-C₆)-alkyl, CONH₂;
or
R8 and R9 together with the C atoms carrying them a 5 to 7 membered, saturated, partially or completely unsaturated ring Cyc2, it being possible for 1 or 2 C atom(s) in the ring also to be replaced by N, O or S, and Cyc2 may optionally be substituted by (C₁-C₆)-alkyl, (C₂-C₅)-alkenyl, (C₂-C₅)-alkynyl, where in each case one CH₂ group may be replaced by O, or substituted by H, F, Cl, OH, CF₃, NO₂, CN, COO(C₁-C₄)-alkyl, CONH₂, CONH(C₁-C₄)-alkyl, OCF₃;
and the pharmaceutically acceptable salts thereof.

2. A compound of the formula I as claimed in claim 1, in which the meanings are
R1 and R2 independently of one another F or H and one of the radicals R1 or R2 = OH, where one of the radicals R1 or R2 must be F;
R3 OH;
R4 OH;
A O or NH;
X C, O or N, where X must be C when Y is S;
Y S or N;
m a number 1 or 2;
R5 hydrogen, F, Cl, Br, I, OH, CF₃, NO₂, CN, COOH, CO(C₁-C₆)-alkyl, COO(C₁-C₆)-alkyl, CONH₂, CONH(C₁-C₆)-alkyl, CON[(C₁-C₆)-alkyl]₂, (C₁-C₆)-alkyl, (C₂-C₆)-alkenyl, (C₂-C₆)-alkynyl, (C₁-C₆)-alkoxy, HO-(C₁-C₆)-alkyl, (C₁-C₆)-alkyl-O-(C₁-C₆)-alkyl, phenyl, benzyl, (C₁-C₄)-alkylcarboxyl, SO-(C₁-C₆)-alkyl, it being possible for one, more than one or all hydrogen(s) in the alkyl or alkoxy radicals to be replaced by fluorine; or
when Y is S, R5 and R6 together with the C atoms carrying them phenyl;
R6 optionally H, (C₁-C₆)-alkyl, (C₁-C₆)-alkenyl, (C₃-C₆)-cycloalkyl, or phenyl that may optionally be substituted by halogen or (C₁-C₄)-alkyl;
B (C₀-C₁₅)-alkanediyl, where one or more C atom(s) in the alkanediyl radical may be replaced independently of one another by -O-, -(C=O)-, -CH=CH-, -C≡C-, -S-, -CH(OH)-, -CHF-, -CF₂-, -(S=O)-, -(SO₂)-, -N((C₁-C₆)-alkyl)-, -N((C₁-C₆)-alkyl-phenyl)- or -NH-;
n a number from 0 to 4;
Cyc1 a 3 to 7 membered saturated, partially saturated or unsaturated ring, where 1 C atom may be replaced by O or S;
R7, R8, R9 hydrogen, F, Cl, Br, I, OH, CF₃, NO₂, CN, COOH, COO(C₁-C₆)-alkyl, CO(C₁-C₄)-alkyl, CONH₂, CONH(C₁-C₆)-alkyl, CON[(C₁-C₆)-alkyl]₂, (C₁-C₆)-alkyl, (C₂-C₆)-alkenyl, (C₂-C₆)-alkynyl, (C₁-C₈)-alkoxy, HO-(C₁-C₆)-alkyl, (C₁-C₆)-alkyl-O-(C₁-C₆)-alkyl, S-(C₁-C₆)-alkyl, SCF₃, SO-(C₁-C₆)-alkyl, it being possible for one, more than one or all hydrogen(s) in the alkyl or alkoxy radicals to be replaced by fluorine;
or
R8 and R9 together with the C atoms carrying them a 5 to 7 membered, saturated, partially or completely unsaturated ring Cyc2, where 1 or 2 C atom(s) in the ring may also be replaced by N, O or S, and Cyc2 may optionally be substituted by (C₁-C₆)-alkyl, (C₂-C₅)-alkenyl, (C₂-C₅)-alkynyl, where in each case one CH₂ group may be replaced by O, or substituted by H, F, Cl, OH, CF₃, NO₂, CN, COO(C₁-C₄)-alkyl, CONH₂, CONH(C₁-C₄)-alkyl, OCF₃.

3. A compound of the formula I as claimed in claim 1 or 2, in which the sugar residues are beta(β)-linked and the stereochemistry in the 2-, 3- and 5-position of the sugar residue has the D-gluco configuration.

4. A compound of the formula I as claimed in claims 1 to 3, in which
R1 and R2 are independently of one another F, H or one of the radicals R1 or R2=OH, where at least one of the radicals R1 or R2 must be F;
R3 is OH;
R4 is OH;
A is O;
X is C, O or N, where X must be C when Y is S;
Y is S or N;
m is a number 1;
R5 is hydrogen, (C₁-C₅)-alkyl, (C₁-C₄)-alkoxy, HO-(C₁-C₄)-alkyl, (C₁-C₄)-alkyl-O-(C₁-C₄)-alkyl, F, Cl, CF₃, OCF₃, OCH₂CF₃, (C₁-C₄)-alkyl-CF₂-, phenyl, benzyl, (C₁-C₄)-alkylcarboxyl, (C₂-C₄)-alkenyl, (C₂-C₄)-alkynyl, COO(C₁-C₄)-alkyl;
or
when Y is S, R5 and R6 together with the C atoms carrying them are phenyl;
R6 is optionally H, (C₁-C₆)-alkyl, (C₁-C₆)-alkenyl, (C₃-C₆)-cycloalkyl, or phenyl that may optionally be substituted by halogen or (C₁-C₄)-alkyl;
B is (C₁-C₄)-alkanediyl, where one CH₂ group may also be replaced by -(C=O)-, -CH(OH)-, -CO-NH-, -CHF-, -CF₂-, -O-;
n is a number 2 or 3;
Cyc1 is an unsaturated 5- or 6-membered ring, where 1 C atom may be replaced by O or S;
R7, R8, R9 are hydrogen, (C₁-C₄)-alkyl, (C₁-C₈)-alkoxy, S-(C₁₋C₄)-alkyl, SCF₃, F, Cl, Br, I, OCF₃, OCH₂CF₃, OH, HO-(C₁-C₄)-alkyl, (C₁-C₄)-alkyl-O-(C₁-C₄)-alkyl, or
R8 and R9 together are -CH=CH-O-, -CH=CH-S-, -CH=CH-CH=CH-, which is optionally substituted by (C₁-C₄)-alkoxy, or -O-(CH₂)ₚ-O-, with p = 1 or 2 and
R7 is hydrogen.

5. A compound of the formula I as claimed in claims 1 to 4, in which
R1, R2 are H or F, where one of the radicals R1, R2 must be F;
R3 is OH;
R4 is OH;
A is O;
X is C and Y is S, or
X is O and Y is N, or
X is N and Y is N;
m is a number 1;
R5 is hydrogen, CF₃, (C₁-C₆)-alkyl, or when Y is S R5 and R6 together with the C atoms carrying them are phenyl;
R6 is optionally H, (C₁-C₄)-alkyl or phenyl;
B is -CH₂-, -C₂H₄-, -C₃H₆, -CO-NH-CH₂- or -CO-CH₂-CH₂-;
n is a number 2 or 3;
Cyc1 is an unsaturated 5 to 6 membered ring, where 1 C atom can be replaced by S;
R7, R8, R9 are hydrogen, (C₁-C₆) alkyl, (C₁-C₄)-alkoxy, S-(C₁₋C₄)-alkyl, SCF₃, F, Cl, Br, I, OCF₃, or
R8 and R9 together are -CH=CH-O-, -CH=CH-CH=CH-, which is optionally substituted by (C₁-C₄)-alkoxy, and
R7 is hydrogen.

6. A compound of the formula I as claimed in claims 1 to 5, in which
R1, R2 are H or F, where one of the radicals R1 or R2 is F;
R3 is OH;
R4 is OH;
A is O;
X is C and Y is S or
X is N and Y is N;
m is a number 1;
R5 is hydrogen, (C₁-C₄)-alkyl or CF₃ or when Y is S R5 and R6 together with the carbon atoms carrying them are phenyl;
R6 is optionally H or (C₁-C₄)-alkyl;
B is -CH₂- or -CO-NH-CH₂-;
n is a number 2 or 3;
Cyc1 is phenyl or thiophene;
R7 is hydrogen, methoxy, F, Cl, Br, I, (C₁-C₄)-alkyl, OCF₃;
R8, R9 are hydrogen or Cl or
R8 and R9 together with the carbon atoms carrying them are phenyl which may optionally be substituted by methoxy, or furan and
R7 is hydrogen.

7. A medicament comprising one or more of the compounds as claimed in one or more of claims 1 to 6.

8. A medicament comprising one or more of the compounds as claimed in one or more of claims 1 to 6 and one or more blood glucose-lowering active ingredients.

9. The use of the compounds as claimed in one or more of claims 1 to 6 for producing a medicament for the treatment of type 1 and type 2 diabetes.

10. The use of the compounds as claimed in one or more of claims 1 to 6 for producing a medicament for lowering blood glucose.

11. The use of the compounds as claimed in one or more of claims 1 to 6 in combination with at least one other blood glucose-lowering , active ingredient for producing a medicament for the treatment of type 1 and type 2 diabetes.

12. The use of the compounds as claimed in one or more of claims 1 to 6 in combination with at least one other blood glucose-lowering active ingredient for producing a medicament for lowering blood glucose.

13. A process for producing a medicament comprising one or more of the compounds as claimed in one or more of claims 1 to 6, which comprises mixing the active ingredient with a pharmaceutically suitable carrier and converting this mixture into a form suitable for administration.

## Revendications

1. Composés de formule I, 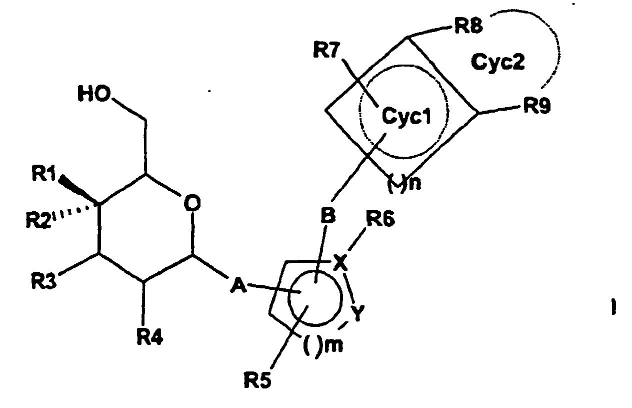 dans laquelle
R1 et R2 signifient, indépendamment l'un de l'autre F, H ou un des radicaux R1 ou R2 signifie OH ;
R3 signifie OH ou F, où au moins un des radicaux R1, R2, R3 doit représenter F ;
R4 signifie OH ;
A signifie O, NH, CH₂, S ou une liaison ;
X signifie C, O, S ou N, où, lorsque Y = O ou S, X doit représenter C ;
Y signifie N, O ou S ;
m signifie un nombre 1 ou 2 ;
R5 signifie hydrogène, F, Cl, Br, I, OH, CF₃, NO₂, CN, COOH, CO(alkyle en C₁ à C₆), COO(alkyle en C₁ à C₆), CONH₂, CONH (alkyle en C₁ à C₆), CON [alkyle en C₁ à C₆]₂, alkyle en C₁ à C₆, alcényle en C₂ à C₆, alcynyle en C₂ à C₆, alcoxy en C₁ à C₆, HO-(alkyle en C₁ à C₆), (alkyle en C₁ à C₆)-O-(alkyle en C₁ à C₆), phényle, benzyle, (alcoxy en C₁ à C₆)carboxyle, où, dans les radicaux alkyle, alcoxy, alcényle ou alcynyle, un, plusieurs ou tous les hydrogènes peuvent être remplacés par fluor ; SO₂-NH₂, SO₂NH (alkyle en C₁ à C₆), SO₂N[alkyle en C₁ à C₆]₂, S-(alkyle en C₁ à C₆), S-(CH₂)ₒ-phényle, SO-(alkyle en C₁ à C₆), SO-(CH₂)ₒ-phényle, SO₂-(alkyle en C₁ à C₆), SO₂-(CH₂)ₒ-phényle, où o = 0 - 6 et le radical phényle peut être jusqu'à disubstitué par F, Cl, Br, OH, CF₃, NO₂, CN, OCF₃, O-(alkyle en C₁ à C₆), (alkyle en C₁ à C₆), MH₂ ;
NH₂, NH-(alkyle en C₁ à C₆), N[alkyle en C₁ à C₆]₂, NH(acyle en C₁ à C₇), phényle, O-(CH₂)ₒ-phényle, où o = 0 - 6, le cycle phényle peut être monosubstitué à trisubstitué par F, Cl, Br, I, OH, CF₃, NO₂, CN, OCF₃, O-(alkyle en C₁ à C₆), alkyle en C₁ à C₆, NH₂, NH (alkyle en C₁ à C₆), N [alkyle en C₁ à C₆]₂, SO₂-CH₃, COOH, COO-(alkyle en C₁ à C₆), CONH₂ ;
ou lorsque Y = S R5 et R6 représentent ensemble avec les atomes de C qui les porte phényle ;
R6 représente le cas échéant H, (alkyle en C₁ à C₆), (alcényle en C₁ à C₆), cycloalkyle en C₃ à C₆, ou phényle, qui peut le cas échéant être substitué par halogène ou alkyle en C₁ à C₄ ;
B signifie (alcane en C₀ à C₁₅)diyle, où un ou plusieurs atomes de carbone du radical alcanediyle peuvent être remplacés indépendamment l'un de l'autre par -O-, -(C=O)-, -CH=CH-, -C≡C-, -S-, -CH(OH)-, -CHF-, -CF₂-, -(S=O)-, -(SO₂)-, -N(alkyle en C₁ à C₆) -, -N((alkyle en C₁ à C₆)phényle)- ou -NH- ;
n signifie un nombre de 0 à 4 ;
Cyc1 signifie un cycle de 3 à 7 chaînons, saturé, partiellement saturé ou insaturé, où 1 atome de carbone peut être remplacé par O, N ou S ;
R7, R8, R9 signifient hydrogène, F, Cl, Br, I, OH, CF₃, NO₂, CN, COOH, COO(alkyle en C₁ à C₆), CO(alkyle en C₁ à C₄), CONH₂, CONH(alkyle en C₁ à C₆), CON[alkyle en C₁ à C₆]₂, alkyle en C₁ à C₆, alcényle en C₂ à C₆, alcynyle en C₂ à C₆, alcoxy en C₁ à C₈, HO-alkyle en C₁ à C₆, (alkyle en C₁ à C₆) -O- (alkyle en C₁ à C₆), où, dans les radicaux alkyle, alcoxy, alcényle ou alcynyle, un, plusieurs ou tous les hydrogènes peuvent être remplacés par fluor ;
SO₂-NH₂, SO₂NH (alkyle en C₁ à C₆), SO₂N [alkyle en C₁ à C₆]₂, S-(alkyle en C₁ à C₆), S-(CH₂)ₒ-phényle, SCF₃, SO- (alkyle en C₁ à C₆), SO-(CH₂)ₒ-phényle, SO₂- (alkyle en C₁ à C₆), SO₂-(CH₂)ₒ-phényle, où o = 0 - 6 et le radical phényle peut être monosubstitué à disubstitué par F, Cl, Br, OH, CF₃, NO₂, CN, OCF₃, O-alkyle en C₁ à C₆, alkyle en C₁ à C₆, NH₂ ;
NH₂, NH- (alkyle en C₁ à C₆), N [alkyle en C₁ à C₆]₂, NH(acyle en C₁ à C₇), phényle, O-(CH₂)ₒ-phényle, où o = 0 - 6, le radical phényle peut être jusqu'à trisubstitué par F, Cl, Br, I, OH, CF₃, NO₂, CN, OCF₃, alcoxy en C₁ à C₈, alkyle en C₁ à C₆, NH₂, NH (alkyle en C₁ à C₆), N[alkyle en C₁ à C₆]₂, SO₂-CH₃, COOH, COO-(alkyle en C₁ à C₆), CONH₂;
ou
R8 et R9 signifient ensemble avec les atomes de carbone qui les portent un cycle Cyc2 de 5 à 7 chaînons, saturé, partiellement ou complètement insaturé, où 1 ou 2 atomes du cycle peuvent également être remplacés par N, O ou S et Cyc2 peut le cas échéant être substitué par alkyle en C₁ à C₆, alcényle en C₂ à C₅, alcynyle en C₂ à C₅, où à chaque fois un groupe CH₂ peut être remplacé par O, ou substitué par H, F, Cl, OH, CF₃, NO₂, CN, COO(alkyle en C₁ à C₄), CONH₂, CONH(alkyle en C₁ à C₄), OCF₃ ;
ainsi que leurs sels pharmaceutiquement acceptables.

2. Composés de formule I selon la revendication 1, dans laquelle
R1 et R2 signifient indépendamment l'un de l'autre F ou H et un des radicaux R1 ou R2 = OH, où un des radicaux R1 ou R2 doit représenter F ;
R3 signifie OH ;
R4 signifie OH ;
A signifie O ou NH ;
X signifie C, O ou N, où, lorsque Y = S, X doit représenter C ;
Y signifie S ou N ;
m signifie un nombre 1 ou 2 ;
R5 signifie hydrogène, F, Cl, Br, I, OH, CF₃, NO₂, CN, COOH, CO(alkyle en C₁ à C₆), COO(alkyle en C₁ à C₆), CONH₂, CONH (alkyle en C₁ à C₆), CON [alkyle en C₁ à C₆]₂, alkyle en C₁ à C₆, alcényle en C₂ à C₆, alcynyle en C₂ à C₆, alcoxy en C₁ à C₆, HO-alkyle en C₁ à C₆, (alkyle en C₁ à C₆)-O-(alkyle en C₁ à C₆), phényle, benzyle, (alkyle en C₁ à C₄) carboxyle, SO-(alkyle en C₁ à C₆), où, dans les radicaux alkyle ou alcoxy, un, plusieurs ou tous les hydrogènes peuvent être remplacés par fluor ; ou lorsque Y = S R5 et R6 signifient, ensemble avec les atomes de carbone qui les portent, phényle ;
R6 signifie le cas échéant H, alkyle en C₁ à C₆, alcényle en C₁ à C₆, cycloalkyle en C₃ à C₆, ou phényle, qui peut le cas échéant être substitué par halogène ou alkyle en C₁ à C₄ ;
B signifie (alcane en C₀ à C₁₅)diyle, où un ou plusieurs atomes de carbone du radical alcanediyle peuvent être remplacés indépendamment l'un de l'autre par -O-, -(C=O)-, -CH=CH-, -C≡C-, -S-, -CH(OH)-, -CHF-, -CF₂-, -(S=O)-, -(SO₂)-, -N(alkyle en C₁ à C₆)-, -N (alkyle en C₁ à C₆-phényl)- ou -NH- ; n signifie un nombre de 0 à 4 ;
Cyc1 signifie un cycle de 3 à 7 chaînons, saturé, partiellement saturé ou insaturé, où un 1 atome de carbone peut être remplacé par O ou S ;
R7, R8, R9 signifient hydrogène, F, Cl, Br, I, OH, CF₃, NO₂, CN, COOH, COO(alkyle en C₁ à C₆), CO(alkyle en C₁ à C₄), CONH₂, CONH (alkyle en C₁ à C₆), CON[alkyle en C₁ à C₆]₂, alkyle en C₁ à C₆, alcényle en C₂ à C₆, alcynyle en C₂ à C₆, alcoxy en C₁ à C₈, HO-alkyle en C₁ à C₆, (alkyle en C₁ à C₆) -O- (alkyle en C₁ à C₆), S-alkyle en C₁ à C₆, SCF₃, SO-alkyle en C₁ à C₆, où, dans les radicaux alkyle ou alcoxy, un, plusieurs ou tous les hydrogènes peuvent être remplacés par fluor ;
ou
R8 et R9 signifient ensemble avec les atomes de carbone qui les portent un cycle Cyc2 de 5 à 7 chaînons, saturé, partiellement ou complètement insaturé, où 1 ou 2 atomes de carbone du cycle peuvent également être remplacés par N, O ou S et Cyc2 peut le cas échéant être substitué par alkyle en C₁ à C₆, alcényle en C₂ à C₅, alcynyle en C₂ à C₅, où à chaque fois un groupe CH₂ peut être remplacé par O ou substitué par H, F, Cl, OH, CF₃, NO₂, CN, COO(alkyle en C₁ à C₄), CONH₂, CONH(alkyle en C₁ à C₄), OCF₃.

3. Composés de formule I selon les revendications 1 ou 2, dans laquelle les radicaux de sucre sont liés en en bêta (β) et la stéréochimie dans la 2ème, 3ème et 5ème position du radical sucre a une configuration D-gluco.

4. Composés de formule I selon les revendications 1 à 3 dans laquelle
R1 et R2 signifient indépendamment l'un de l'autre F, H ou un des radicaux R1 ou R2 = OH, où au moins un des radicaux R1 ou R2 doit représenter F ;
R3 signifie OH ;
R4 signifie OH ;
A signifie O ;
X signifie C, O ou N, où, lorsque Y = S, X doit représenter C ;
Y signifie S ou N ;
m signifie un nombre 1 ;
R5 signifie hydrogène ; alkyle en C₁ à C₅, alcoxy en C₁ à C₄, HO-alkyle en C₁ à C₄, (alkyle en C₁ à C₄) -O- (alkyle en C₁ à C₄), F, Cl, CF₃, OCF₃, OCH₂CF₃, (alkyle en C₁ à C₄) -CF₂-, phényle, benzyle, (alkyle en C₁ à C₄) carboxyle, alcényle en C₂ à C₄, alcynyle en C₂ à C₄, COO(alkyle en C₁ à C₄);
ou
lorsque Y = S, R5 et R6 signifient ensemble avec les atomes de carbone qui les portent phényle ;
R6 signifie le cas échéant H, alkyle en C₁ à C₆, alcényle en C₁ à C₆, cycloalkyle en C₃ à C₆, ou phényle, qui peut le cas échéant être substitué par halogène ou alkyle en C₁ à C₄ ;
B signifie (alcane en C₁ à C₄)diyle, où un groupe CH₂- peut également être remplacé par -(C=O)-, -CH(OH)-, -CO-NH-, -CHF-, -CF₂-, -O- ;
n signifie un nombre 2 ou 3 ;
Cyc1 signifie un cycle insaturé de 5 à 6 chaînons, où 1 atome de carbone peut être remplacé par O ou S ;
R7, R8, R9 signifient hydrogène, alkyle en C₁ à C₄, alcoxy en C₁ à C₈, S-(alkyle en C₁ à C₄), SCF₃, F, Cl, Br, I, OCF₃, OCH₂CF₃, OH, HO-alkyle en C₁ à C₄, (alkyle en C₁ à C₄)-O-(alkyle en C₁ à C₄), ou
R8 et R9 signifient ensemble -CH=CH-O-, -CH=CH-S-, -CH=CH-CH=CH-, qui est le cas échéant substitué par alcoxy en C₁ à C₄, ou -O-(CH₂)ₚ-O-, avec p = 1 ou 2 et R7 signifie hydrogène.

5. Composés de formule I selon les revendications 1 à 4, dans laquelle
R1, R2 signifient H ou F, où un des radicaux R1, R2 doit représenter F ;
R3 signifie OH ;
R4 signifie OH ;
A signifie O ;
X signifie C et Y = S, ou
X signifie O et Y = N, ou
X signifie N et Y = N ;
m signifie un nombre 1 ;
R5 signifie hydrogène, CF₃, (alkyle en C₁ à C₆), ou lorsque Y = S R5 et R6 représentent ensemble avec les atomes de carbone qui les portent phényle ;
R6 signifie le cas échéant H, alkyle en C₁ à C₄ ou phényle ;
B signifie -CH₂-, -C₂H₄-, -C₃H₆, -CO-NH-CH₂- ou -CO-CH₂-CH₂- ;
n signifie un nombre 2 ou 3 ;
Cyc1 signifie un cycle insaturé de 5 à 6 chaînons, 1 atome de carbone pouvant être remplacé par S ;
R7, R8, R9 signifient hydrogène, alkyle en C₁ à C₆, alcoxy en C₁ à C₄, S-(alkyle en C₁ à C₄), SCF₃, F, Cl, Br, I, OCF₃ ou
R8 et R9 signifient ensemble -CH=CH-O-, -CH=CH-CH=CH-, qui est le cas échéant substitué par alcoxy en C₁ à C₄, et
R7 signifie hydrogène.

6. Composés de formule I selon les revendications 1 à 5, dans laquelle
R1, R2 signifient H ou F, où un des radicaux R1 ou R2 signifie F ;
R3 signifie OH ;
R4 signifie OH ;
A signifie O ;
X signifie C et Y = S ou
X signifie N et Y = N ;
m signifie un nombre 1 ;
R5 signifie hydrogène, alkyle en C₁ à C₄ ou CF₃ ou, lorsque Y = S R5 et R6 représentent ensemble avec les atomes de carbone qui les portent phényle ;
R6 signifie le cas échéant H ou alkyle en C₁ à C₄ ;
B signifie -CH₂- ou -CO-NH-CH₂- ; n signifie un nombre 2 ou 3 ;
Cyc1 signifie phényle ou thiophène ;
R7 signifie hydrogène, méthoxy, F, Cl, Br, I, alkyle en C₁ à C₄, OCF₃ ;
R8, R9 signifient hydrogène ou Cl ou
R8 et R9 signifient ensemble avec les atomes de carbone qui les portent phényle, qui peut le cas échéant être substitué par méthoxy, ou furanne et
R7 signifie hydrogène.

7. Médicament contenant un ou plusieurs des composés selon l'une ou plusieurs des revendications 1 à 6.

8. Médicament contenant un ou plusieurs des composés selon l'une ou plusieurs des revendications 1 à 6 et une ou plusieurs substances actives abaissant la glycémie.

9. Utilisation des composés selon l'une ou plusieurs des revendications 1 à 6 pour la préparation d'un médicament destiné au traitement du diabète de type 1 et de type 2.

10. Utilisation des composés selon l'une ou plusieurs des revendications 1 à 6 pour la préparation d'un médicament destiné à abaisser la glycémie.

11. Utilisation des composés selon l'une ou plusieurs des revendications 1 à 6 en combinaison avec au moins une autre substance active abaissant la glycémie pour la préparation d'un médicament destiné au traitement du diabète de type 1 et de type 2.

12. Utilisation des composés selon l'une ou plusieurs des revendications 1 à 6 en combinaison avec au moins une autre substance active abaissant la glycémie pour la préparation d'un médicament destiné à abaisser la glycémie.

13. Procédé pour la préparation d'un médicament contenant un ou plusieurs des composés selon l'une ou plusieurs des revendications 1 à 6, **caractérisé en ce que** la substance active est mélangée avec un support pharmaceutiquement approprié et ce mélange est amené dans une forme appropriée pour l'administration.
